(19) Europäisches Patentamt / European Patent Office / Office européen des brevets

(11) **EP 4 656 654 A1**

(12) **EUROPEAN PATENT APPLICATION**
published in accordance with Art. 153(4) EPC


(43) Date of publication:
**03.12.2025 Bulletin 2025/49**

(21) Application number: **24850373.2**

(22) Date of filing: **11.09.2024**

(51) International Patent Classification (IPC):
***C07K 14/655*** $^{(2006.01)}$ ***A61K 38/31*** $^{(2006.01)}$
***A61P 35/00*** $^{(2006.01)}$ ***A61P 5/00*** $^{(2006.01)}$
***A61K 38/08*** $^{(2019.01)}$

(52) Cooperative Patent Classification (CPC):
**A61K 38/08; A61K 38/31; A61P 5/00; A61P 35/00; C07K 14/655**

(86) International application number:
**PCT/CN2024/118116**

(87) International publication number:
**WO 2025/066894 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 CN 202311262443**



(71) Applicant: **Qianhangjiang Pharmaceutical Co., Ltd.**
**Jiaxing, Zhejiang 314408 (CN)**

(72) Inventors:
- **QIAN, Naifeng**
  **Jiaxing, Zhejiang 314408 (CN)**
- **JIANG, Xuliang**
  **Jiaxing, Zhejiang 314408 (CN)**

(74) Representative: **Valet Patent Services Limited**
**c/o Caya 83713X**
**Am Börstig 5**
**96052 Bamberg (DE)**


(54) **SOMATOSTATIN PEPTIDE ANALOGUE, CHELATE AND USE THEREOF**


(57) The present invention relates to novel structures, synthesis methods, and applications of human somatostatin analogs comprising either a cyclic or non-cyclic hexapeptide unit, wherein the amino acid residues at positions 2 to 5 are represented by -$X_1$-(D/L)-Trp-$X_2$-$X_3$-, or a cyclic or non-cyclic octapeptide unit, wherein the amino acid residues at positions 2 to 7 are represented by -Cys-$X_1$-(D/L)-Trp-$X_2$-$X_3$-Cys-. In these sequences, $X_1$ is an $\alpha$-amino acid residue containing an aromatic group on the C$\alpha$ side chain, $X_2$ is a Lys derivative, and $X_3$ is an $\alpha$-amino acid residue. The Lys derivative has the following structure:

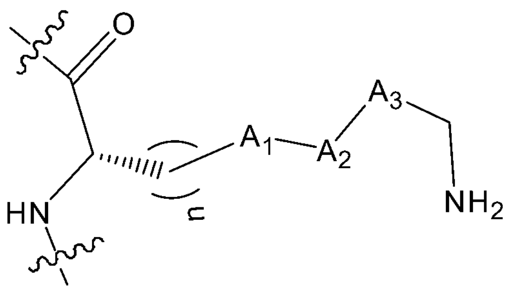

wherein n is 0 or 1; $A_1$, $A_2$, and $A_3$ are independently selected from $C(R)_2$, O, S, NR, C=O, C=S, C=NR, and $C=C(R)_2$. The present invention challenges the conventional understanding that modification of the Lys$^9$ residue in somatostatin significantly reduces biological activity. By altering the Lys$^9$ residue in somatostatin analogs, the present invention provides analogs with enhanced or comparable binding affinity to SSTR2, thereby improving their therapeutic efficacy.


EP 4 656 654 A1

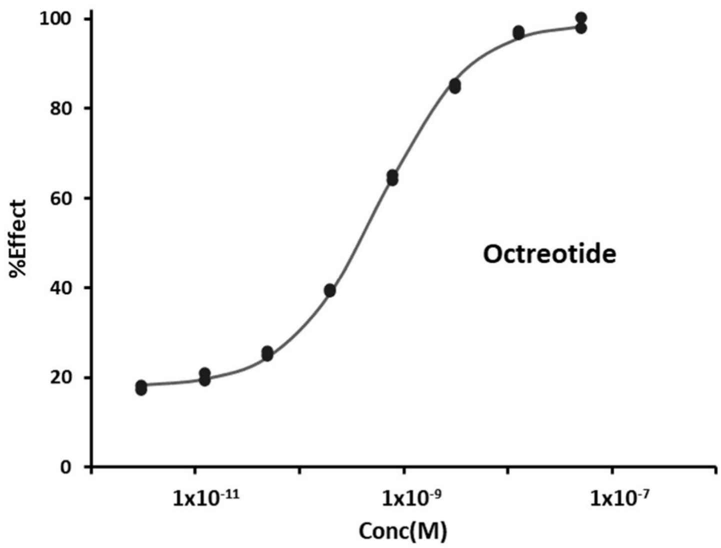


FIG. 1

## Description

Background of the Invention

Natural human somatostatins (SSTs) are cyclic peptides, including somatostatin-14 (SST-14) and somatostatin-28 (SST-28), which consist of 14 and 28 amino acids, respectively. These peptides bind to somatostatin receptors (SSTRs) expressed on the surface of target cells, leading to various biological effects, including the inhibition of hormone secretion. SSTs play a critical role in regulating the human endocrine system by modulating the secretion of insulin, glucagon, thyroid-stimulating hormone, growth hormone, prolactin, and cholecystokinin, among others.

Somatostatin receptors (SSTRs) belong to the G protein-coupled receptors (GPCRs) that signal through the inhibitory Gi/o family of G proteins. The binding of somatostatins or somatostatin analogs (SSAs) to SSTRs triggers an intracellular signaling cascade that inhibits the release of secretory hormone vesicles. The SSTR family comprises five distinct subtypes: SSTR1, SSTR2, SSTR3, SSTR4, and SSTR5, each exhibiting unique tissue distribution and physiological functions. Among these, SSTR2 is frequently overexpressed in neuroendocrine tumors (NETs), making it a primary target for somatostatin receptor agonists. Due to these receptor-binding characteristics, SSAs have been widely utilized in clinical applications for both diagnostic imaging and therapeutic interventions in diseases characterized by hormone secretion disorders, such as carcinoid syndrome, gigantism, acromegaly, hyperthyroidism, and Cushing's disease.

Currently, all U.S. Food and Drug Administration (FDA)-approved somatostatin receptor agonists are peptide analogs of SST-14, including octreotide and lanreotide, which primarily target SSTR2, and pasireotide, which exhibits broader receptor binding by targeting SSTR1, SSTR2, SSTR3, and SSTR5. Numerous studies have demonstrated that different SSTR subtypes play crucial roles in various tumor types beyond NETs, necessitating the development of more selective pharmacological agents to investigate and exploit subtype-specific functions.

In the present invention, natural somatostatin is referred to as a tetradecapeptide (Formula I) with the following structure:

```
     1   2   3   4   5   6   7   8   9  10  11  12  13  14
H-Ala-Gly-Cys-Lys-Asn-Phe-Phe-Trp-Lys-Thr-Phe-Thr-Ser-Cys-OH
          |_____________________________________________|
```

Formula I

The somatostatin analogs disclosed in the present invention encompass both linear and cyclic peptide derivatives of SST-14, wherein one or more amino acid residues in the sequence of Formula I are substituted, deleted, or replaced with other amino acids, or wherein one or more functional groups are modified. These analogs retain high affinity for at least one somatostatin receptor subtype, generally within the nanomolar range.

Due to the extremely short half-life of natural somatostatin, its clinical application is limited. To address this limitation, extensive research efforts in the 1970s led to the synthesis of numerous somatostatin analogs. These studies identified the key functional role of $Trp^8$-$Lys^9$ in receptor binding and laid the foundation for the clinical development of SSAs. One approach, pioneered by a Sandoz research team, involved the use of a hexapeptide core structure (Cys-Phe-DTrp-Lys-Thr-Cys) and the systematic extension of both the N- and C-termini. In 1980, this work led to the successful synthesis of Octreotide, a highly stable and biologically active somatostatin analog with the following structure:

H-DPhe-Cys-Phe-DTrp-Lys-Thr-Cys-Thr-ol

wherein, unless otherwise specified, amino acid residues are in the L-form, and amino acid residues in the D-form are designated with the prefix "D".

Due to its demonstrated superior efficacy in inhibiting growth hormone secretion and sufficient in vivo stability over natural somatostatins, Octreotide was selected for clinical trials. In 1988, the U.S. Food and Drug Administration (FDA) approved it for the treatment of acromegaly and carcinoid tumors. Additionally, the Octreotide derivative DOTA-TATE([$Tyr^3$]-octreotide) has been conjugated with positron-emitting isotope metal ions to form a radiolabeled complex for imaging SSTR-overexpressing tumors. Furthermore, DOTA-TATE has been developed into a radiopharmaceutical therapy, wherein its coupling with $\beta$-emitting isotope metal ions enables the targeted destruction of tumor cells.

Several other somatostatin analogs have also been developed and marketed, including Lanreotide, Vapreotide, and Pasireotide. A large body of research has demonstrated that $Trp^8$-$Lys^9$ forms the core binding motif of SST-14 to its receptors and that these two amino acid residues are essential for biological activity. Consequently, they are recognized as key pharmacophores in somatostatin analogs.

Despite efforts to modify the $Lys^9$ residue to enhance receptor binding and biological activity, most modifications have failed to improve affinity or efficacy. Instead, alterations to $Lys^9$ frequently result in a substantial loss of receptor

affinity and biological function. Prior studies have established that $Lys^9$ is essential, as substitutions, even with structurally similar residues such as arginine (Arg) or ornithine (Orn), lead to a significant reduction in biological activity.

Definitions

Unless otherwise defined, all technical and scientific terms used herein have the meanings commonly understood by those skilled in the art to which the present invention pertains.

The abbreviations used in the present invention are defined as follows:

RT = room temperature; MS = mass spectrometry; Min = minute; HPLC = high-performance liquid chromatography; UPLC = ultra-high-performance liquid chromatography; LC-MS = liquid chromatography-mass spectrometry; MW = molecular weight; $N_2$ = nitrogen gas; $I_2$ = iodine; VC = vitamin C; $EC_{50}$ = half-maximal effective concentration; DMF = dimethylformamide; TFA = trifluoroacetic acid; BOC = tert-butyloxycarbonyl; Fmoc = 9-fluorenylmethoxycarbonyl; HOBT = hydroxybenzotriazole; DODT = 3,6-dioxa-1,8-octanedithiol; AcOH = acetic acid; DCM = dichloromethane; ACN = acetonitrile; DOTA = 1,4,7,10-tetraazacyclododecane-1,4,7,10-tetraacetic acid; TETA = 1,4,8,11-tetraazacyclotetradecane-1,4,8,11-tetraacetic acid; EDTA = ethylenediaminetetraacetic acid; DTPA = diethylenetriaminepentaacetic acid; EA = ethyl acetate.

Amino acid abbreviations are defined as follows:

Cys = cysteine; Trp = tryptophan; Lys = lysine; Tyr = tyrosine; Ser = serine; Ala = alanine; Asp = aspartic acid; Glu = glutamic acid; Gly = glycine; His = histidine; Ile = isoleucine; Leu = leucine; Met = methionine; Asn = asparagine; Phe = phenylalanine; Pro = proline; Gln = glutamine; Arg = arginine; Thr = threonine; Val = valine; Phg = phenylglycine; Orn = ornithine; DTrp = (D-)tryptophan; DPhe = (D-)phenylalanine.

Unless otherwise specified, amino acid residues are in the L-configuration.

Detailed Description of the Invention

A series of structural studies on the SSTR2 protein reveal that the binding pocket of SSTR2 for $Lys^9$ in somatostatin or somatostatin analogs (SSAs) provides sufficient space to accommodate additional atoms when bound by $Lys^9$. Therefore, the $Lys^9$ side chain is modified to fit more tightly into the pocket, thereby reducing the number of possible conformations of the $Lys^9$ side chain. These modifications enhance the binding interactions between the drug molecules and SSTR2, thereby improving both binding affinity and therapeutic efficacy.

Accordingly, an object of the present invention is to provide a somatostatin analog, or a pharmaceutically acceptable salt, complex, or chelate thereof, wherein the somatostatin analog comprises a cyclic or non-cyclic hexapeptide unit having a residue sequence at positions 2 to 5 represented by -$X_1$-(D/L)-Trp-$X_2$-$X_3$-, or a cyclic or non-cyclic octapeptide unit having a residue sequence at positions 2 to 7 represented by -Cys-$X_1$-(D/L)-Trp-$X_2$-$X_3$-Cys-. In these sequences:

- $X_1$ is an $\alpha$-amino acid residue containing an aromatic group on the $C\alpha$ side chain;
- $X_3$ is an $\alpha$-amino acid residue;
- $X_2$ is a Lys derivative having the following structure:

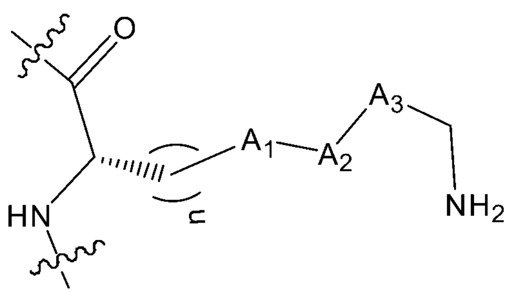

Wherein n is 0 or 1;

$A_1$, $A_2$, and $A_3$ are each independently selected from $C(R)_2$, O, S, NR, C=O, C=S, C=NR, and $C=C(R)_2$, wherein R is selected from H, $R_1$, $-R'OR_1$, $-R'SR_1$, $-R'N(R_1)_2$, $-R'C(O)R_1$, $-R'S(O)R_1$, $- R'S(O)_2R_1$, $-R'C(O)N(R_1)_2$, $-R'N(R_1)C(O)R_4$, $-R'C(O)OR_1$, and $-R'OC(O)R_1$, wherein R' and $R_1$ are further defined as follows:

- R' is selected from H, $C_1$-$C_6$ alkylene, $C_2$-$C_6$ alkenylene, $C_2$-$C_6$ alkynylene, $C_3$-$C_8$ cycloalkylene, $C_3$-$C_8$ cycloalkenylene, halogenated $C_1$-$C_6$ alkylene, halogenated $C_2$-$C_6$ alkenylene, halogenated $C_2$-$C_6$ alkynylene,

halogenated $C_3$-$C_8$ cycloalkylene, halogenated $C_3$ - $C_8$ cycloalkenylene, ($C_6$-$C_8$) arylene, a 3- to 8-membered heterocyclylene containing 1 to 3 heteroatoms selected from N, O, and S, or a 3- to 8-membered heteroarylene containing 1 to 3 heteroatoms selected from N, O, and S; and

- $R_1$ is selected from H, halogen, CN, $NO_2$, $C_1$-$C_6$ alkyl, $C_2$-$C_6$ alkenyl, $C_2$-$C_6$ alkynyl, $C_3$-$C_8$ cycloalkyl, $C_3$-$C_8$ cycloalkenyl, halogenated $C_1$-$C_6$ alkyl, halogenated $C_2$-$C_6$ alkenyl, halogenated $C_2$-$C_6$ alkynyl, halogenated $C_3$-$C_8$ cycloalkyl, halogenated $C_3$-$C_8$ cycloalkenyl, ($C_6$-$C_8$) aryl, a 3- to 8-membered heterocyclic group containing 1 to 3 heteroatoms selected from N, O, and S, or a 3- to 8-membered heteroaryl group containing 1 to 3 heteroatoms selected from N, O, and S ;

R in $C(R)_2$ may form a 3- to 8-membered carbon ring with R in its own $C(R)_2$, with R in an adjacent $C(R)_2$, or with R in a $C(R)_2$ separated by one $C(R)_2$. Additionally, R in $C(R)_2$ may form a 3- to 8-membered heterocyclic ring containing 1 to 3 heteroatoms selected from N, O, and S ;
When n is 0, R in $C(R)_2$ at positions $A_1$, $A_2$, and $A_3$ are not simultaneously H ;
$A_2$ may form a double or triple bond with either $A_1$ or $A_3$ ;
As used herein, the phrase "R in $C(R)_2$ forms a 3- to 8-membered carbon ring with R in its own $C(R)_2$, with R in an adjacent $C(R)_2$, or with R in a $C(R)_2$ separated by one $C(R)_2$, or forms a 3- to 8-membered heterocyclic ring containing 1 to 3 heteroatoms selected from N, O, and S" encompasses the following four configurations:

- The two R groups in $C(R)_2$ at each individual position $A_1$, $A_2$, or $A_3$ form a 3- to 8-membered carbon ring or a 3- to 8-membered heterocyclic ring containing 1 to 3 heteroatoms selected from N, O, and S ;
- R in $C(R)_2$ at position $A_1$ and R in $C(R)_2$ at position $A_3$ are connected to form a ring, such that $A_1$, $A_2$, and $A_3$ together form a 3- to 8-membered carbon ring or a 3- to 8-membered heterocyclic ring containing 1 to 3 heteroatoms selected from N, O, and S ;

- R in $C(R)_2$ at position $A_1$ and R in $C(R)_2$ at position $A_2$ are connected to form a ring, such that $A_1$ and $A_2$ together form a 3- to 8-membered carbon ring or a 3- to 8-membered heterocyclic ring containing 1 to 3 heteroatoms selected from N, O, and S ;
- R in $C(R)_2$ at position $A_2$ and R in $C(R)_2$ at position $A_3$ are connected to form a ring, such that $A_2$ and $A_3$ together form a 3- to 8-membered carbon ring or a 3- to 8-membered heterocyclic ring containing 1 to 3 heteroatoms selected from N, O, and S.In a preferred embodiment, the somatostatin analog comprises a cyclic or non-cyclic hexapeptide unit, wherein the residue sequence at positions 1 to 6 of the hexapeptide unit is represented by: Xa-$X_1$-(D/L-)Trp-$X_2$-$X_3$-Xb; the somatostatin analog may further comprise a cyclic or non-cyclic octapeptide unit, wherein the residue sequence at positions 1 to 8 of the octapeptide unit is represented by: $X_0$-Cys-$X_1$-(D/L-)Trp-$X_2$-$X_3$-Cys-Xb, wherein Xa and Xb are amino acid residues, and $X_0$ is an $\alpha$-amino acid residue comprising an aromatic group on the side chain.

In a preferred embodiment, $X_1$ is a Phe, Tyr, Trp, or Phg (phenylglycine, phenylglycine amide) residue, and $X_3$ is a Thr, Ser, Cys, or tyrosylbenzyl residue.
In a preferred embodiment, the somatostatin analog has a cyclic octapeptide unit structure as shown in the following:

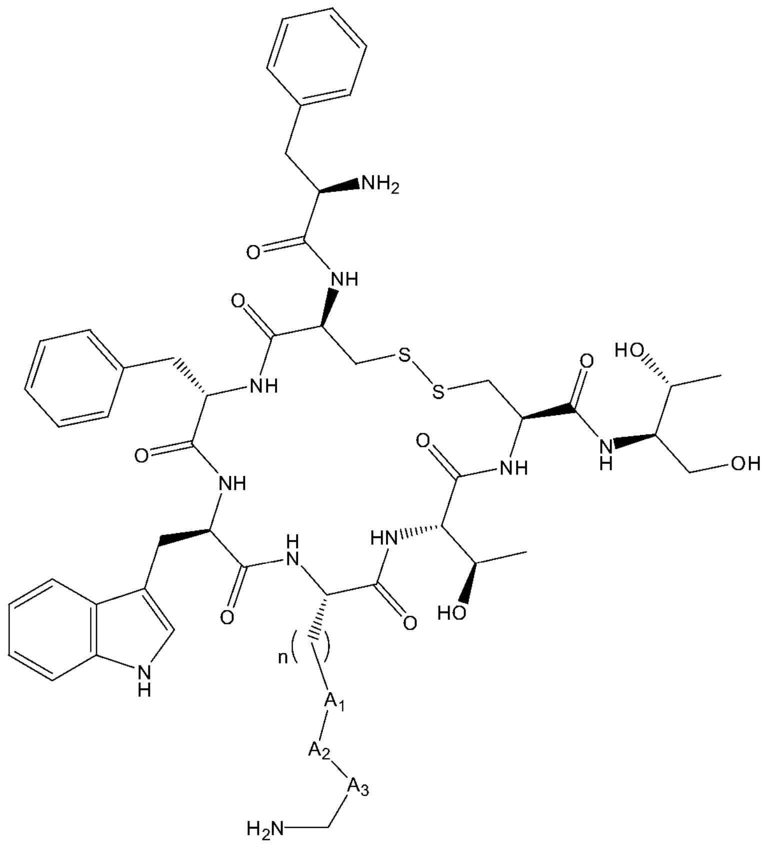

Formula II

Wherein n is 0 or 1;

$A_1$, $A_2$, and $A_3$ are each independently selected from $C(R)_2$, O, S, NR, C=O, C=S, C=NR, and $C=C(R)_2$, wherein R is selected from H, $R_1$, -R'O$R_1$, -R'S$R_1$, -R'N$(R_1)_2$, -R'C(O)$R_1$, -R'S(O)$R_1$, - R'S$(O)_2R_1$, -R'C(O)N$(R_1)_2$, -R'N$(R_1)$C(O)$R_1$, -R'C(O)O$R_4$, and -R'OC(O)$R_1$ , wherein R' and $R_1$ are further defined as follows:

- R' is selected from H, $C_1$-$C_6$ alkylene, $C_2$-$C_6$ alkenylene, $C_2$-$C_6$ alkynylene, $C_3$-$C_8$ cycloalkylene, $C_3$-$C_8$ cycloalkenylene, halogenated $C_1$-$C_6$ alkylene, halogenated $C_2$-$C_6$ alkenylene, halogenated $C_2$-$C_6$ alkynylene, halogenated $C_3$-$C_8$ cycloalkylene, halogenated $C_3$-$C_8$ cycloalkenylene, ($C_6$-$C_8$) arylene, 3- to 8-membered heterocyclylene with 1 to 3 heteroatoms selected from N, O, and S, or 3- to 8-membered heteroarylene with 1 to 3 heteroatoms selected from N, O, and S; and
- $R_1$ is selected from H, halogen, CN, $NO_2$, $C_1$-$C_6$ alkyl, $C_2$-$C_6$ alkenyl, $C_2$-$C_6$ alkynyl, $C_3$-$C_8$ cycloalkyl, $C_3$-$C_8$ cycloalkenyl, halogenated $C_1$-$C_6$ alkyl, halogenated $C_2$-$C_6$ alkenyl, halogenated $C_2$-$C_6$ alkynyl, halogenated $C_3$-$C_8$ cycloalkyl, halogenated $C_3$-$C_8$ cycloalkenyl, ($C_6$-$C_8$) aryl, 3- to 8-membered heterocyclic groups with 1 to 3 heteroatoms selected from N, O, and S, or 3- to 8-membered heteroaryl groups with 1 to 3 heteroatoms selected from N, O, and S;

$A_2$ may form a double bond or a triple bond with either $A_1$ or $A_3$ ;

R in $C(R)_2$ may form a 3- to 8-membered carbon ring with R in its own $C(R)_2$, with R in an adjacent $C(R)_2$, or with R in a $C(R)_2$ separated by one $C(R)_2$, or may form a 3- to 8-membered heterocyclic ring with 1 to 3 heteroatoms selected from N, O, and S ;

When n is 0, R in $C(R)_2$ at positions $A_1$, $A_2$, and $A_3$ are not simultaneously H ;

In a preferred embodiment, the R group in $C(R)_2$ at position $A_1$ and the R group in $C(R)_2$ at position $A_3$ are linked to form a ring, such that $A_1$, $A_2$, and $A_3$ together form a 3- to 8-membered carbocyclic or heterocyclic ring, wherein the heterocyclic ring comprising 1 to 3 heteroatoms selected from N, O, and S.

Preferably, the R group in $C(R)_2$ at position $A_1$ and the R group in $C(R)_2$ at position $A_3$ are linked to form a ring, such that $A_1$, $A_2$, and $A_3$ together form a 5- to 7-membered carbocyclic or heterocyclic ring, wherein the heterocyclic ring comprising 1 to 2 heteroatoms selected from N, O, and S.

In a preferred embodiment, $A_2$ forms a double or triple bond with either $A_1$ or $A_3$.

In a preferred embodiment, R' is a connecting bond, or selected from $C_1$-$C_4$ alkylene, $C_2$-$C_4$ alkenylene, $C_2$-$C_4$ alkynylene, $C_3$-$C_7$ cycloalkylene, $C_3$-$C_7$ cycloalkenylene, halogenated $C_1$-$C_4$ alkylene, halogenated $C_2$-$C_4$ alkenylene, halogenated $C_2$-$C_4$ alkynylene, halogenated $C_3$-$C_7$ cycloalkylene, halogenated $C_3$-$C_7$ cycloalkenylene, ($C_6$-$C_8$) arylene, a 3- to 7-membered heterocyclic group optionally comprising 1 to 3 heteroatoms selected from N, O, and S, or a 3- to 7-membered heteroaryl group optionally comprising 1 to 3 heteroatoms selected from N, O, and S ; and

$R_1$ is selected from H, halogen, CN, $NO_2$, $C_1$-$C_4$ alkyl, $C_2$-$C_4$ alkenyl, $C_2$-$C_4$ alkynyl, $C_3$-$C_7$ cycloalkyl, $C_3$-$C_7$ cycloalkenyl, halogenated $C_1$-$C_4$ alkyl, halogenated $C_2$-$C_4$ alkenyl, halogenated $C_2$-$C_4$ alkynyl, halogenated $C_3$-$C_7$ cycloalkyl, halogenated $C_3$-$C_7$ cycloalkenyl, ($C_6$-$C_8$) aryl, a 3- to 7-membered heterocyclic group optionally comprising 1 to 3 heteroatoms selected from N, O, and S, or a 3- to 7-membered heteroaryl group optionally comprising 1 to 3 heteroatoms selected from N, O, and S.

In a preferred embodiment, R' is a connecting bond or is selected from C1-C3 alkylene, C2-C3 alkenylene, C2-C3 alkynylene, C5-C6 cycloalkylene, C5-C6 cycloalkenylene, halogenated C1-C3 alkylene, halogenated C2-C3 alkenylene, halogenated C2-C3 alkynylene, halogenated C5-C6 cycloalkylene, halogenated C5-C6 cycloalkenylene, (C6-C8)arylene, 5-6 membered heterocyclylene with 1-2 heteroatoms selected from N, O, S, 5-6 membered heteroarylene with 1-2 heteroatoms selected from N, O, S; and

R1 is selected from H, halogen, CN, NO2, C1-C3 alkyl, C2-C3 alkenyl, C2-C3 alkynyl, C5-C6 cycloalkyl, C5-C6 cycloalkenyl, halogenated C1-C3 alkyl, halogenated C2-C3 alkenyl, halogenated C2-C3 alkynyl, halogenated C5-C6 cycloalkyl, halogenated C5-C6 cycloalkenyl, (C6-C8)aryl, 5-6 membered heterocyclic group having 1 to 2 heteroatoms selected from N, O, and S, and 5-6 membered heteroaryl having 1 to 2 heteroatoms selected from N, O, and S.

In a preferred embodiment, $X_2$ is a residue of a Lys derivative represented by the following structure:

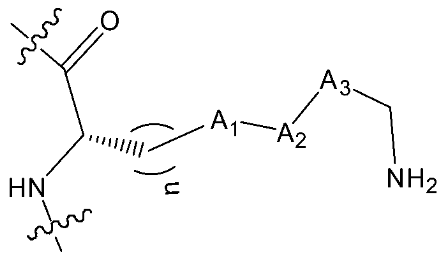

It is selected from:

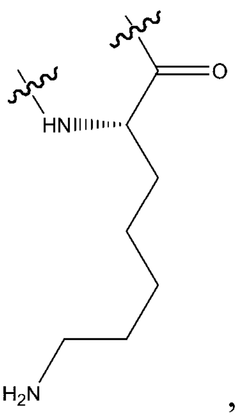
,
Lys(a) residue

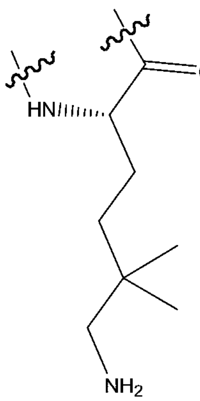
,
Lys(b) residue

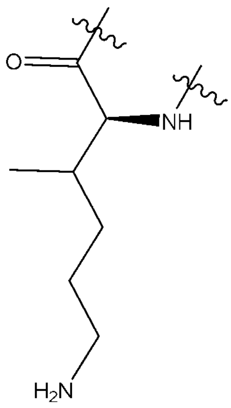
,
Lys(c) residue

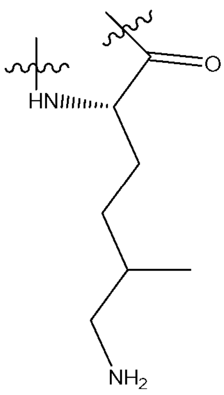
,
Lys(d) residue

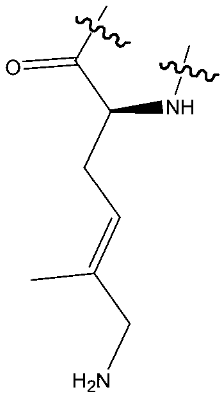 ,
Lys(e) residue

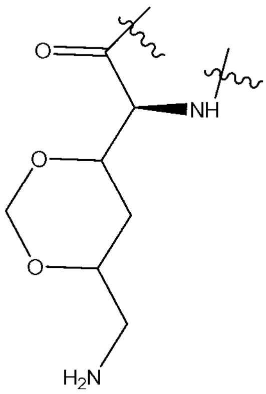 ,
Lys(f) residue

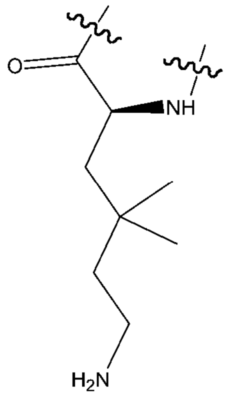 ,
Lys(g) residue

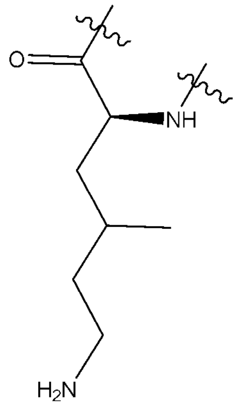 ,
Lys(h) residue

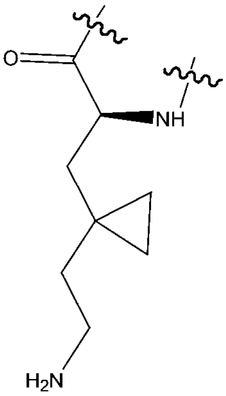 ,
Lys(i) residue

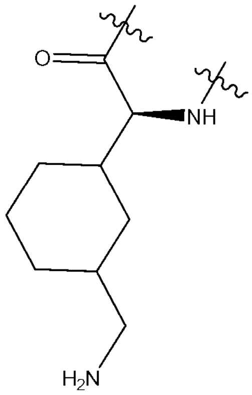 ,
Lys(j) residue

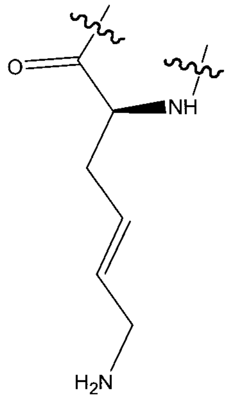 ,
Lys(k) residue

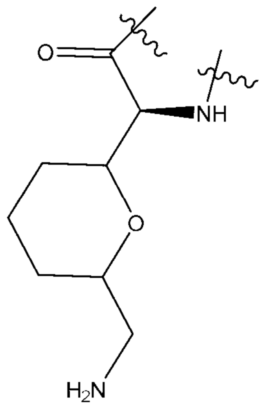 ,
Lys(l) residue

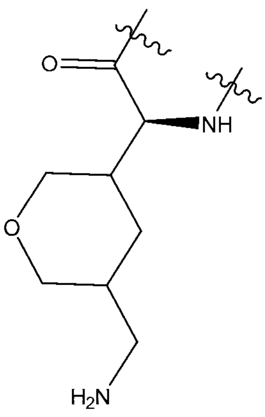 ,
Lys(m) residue

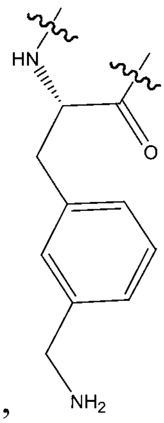 ,
Lys(n) residue

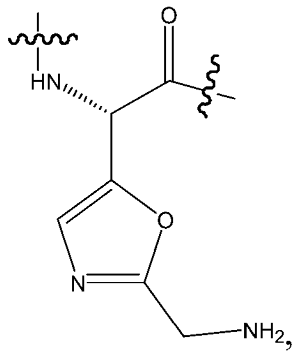
Lys(o) residue

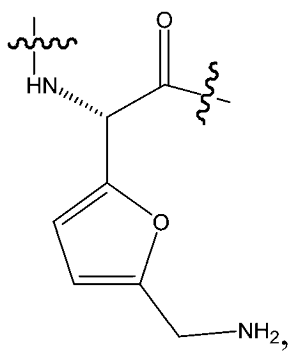
Lys(p) residue

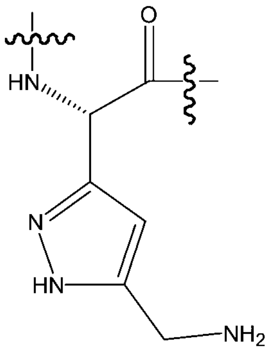,
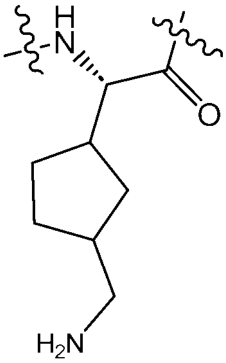,
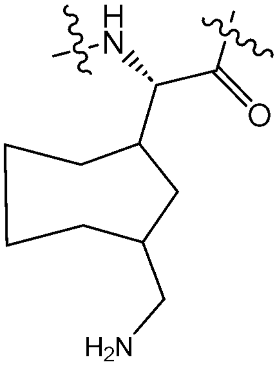

Lys(q) residue Lys(r) residue Lys(s) residue

Among these, Lys(c), Lys(d), Lys(f), Lys(h), Lys(j), Lys(l), Lys(m), Lys(r), and Lys(s) may be any stereoisomers of their respective side chain. For example, Lys(d) may exist as either of the following two stereoisomers:

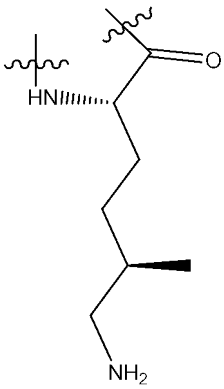,
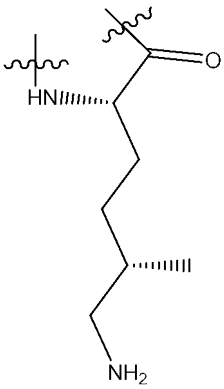.

Lys(j) may be any of the following four stereoisomers:

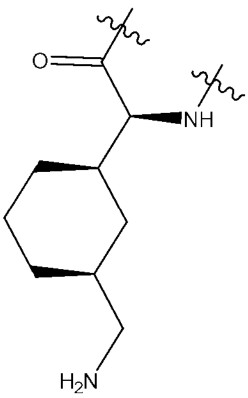,
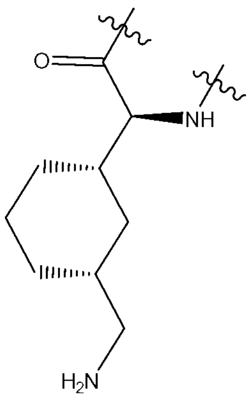,
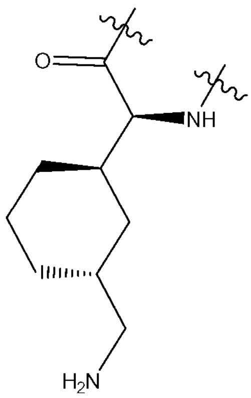,
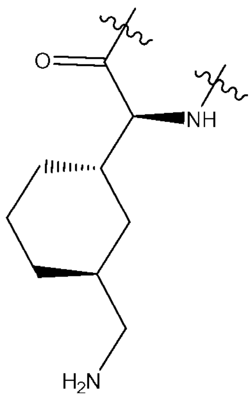.

In a preferred embodiment, the novel somatostatin analog of the present invention has one of the following structures:

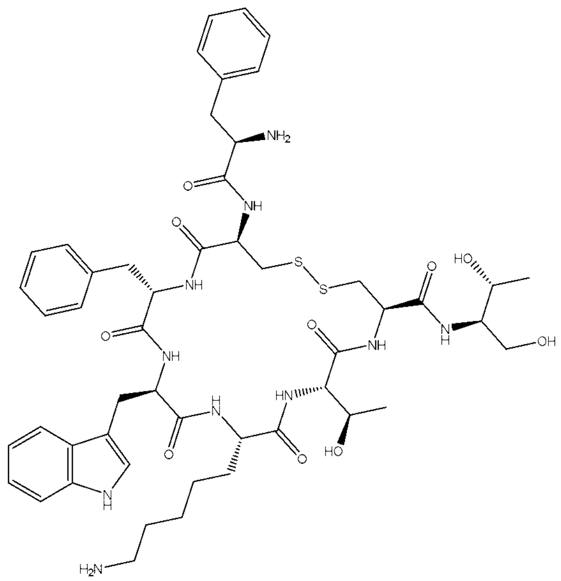

core amino acid sequence Phe-DTrp-Lys(a) -Thr

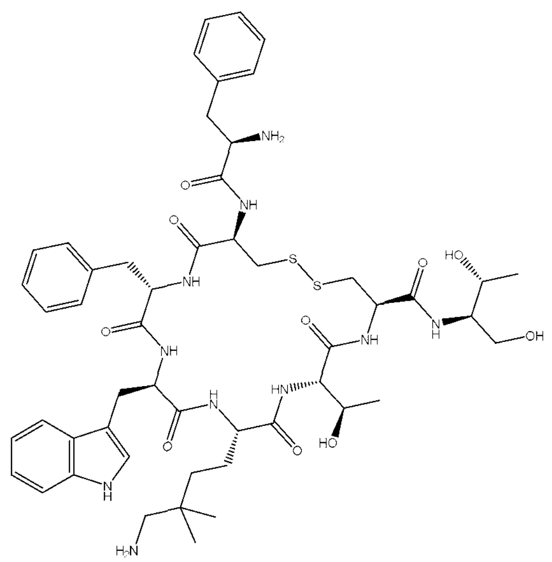

core amino acid sequence Phe-DTrp-Lys(b)-Thr

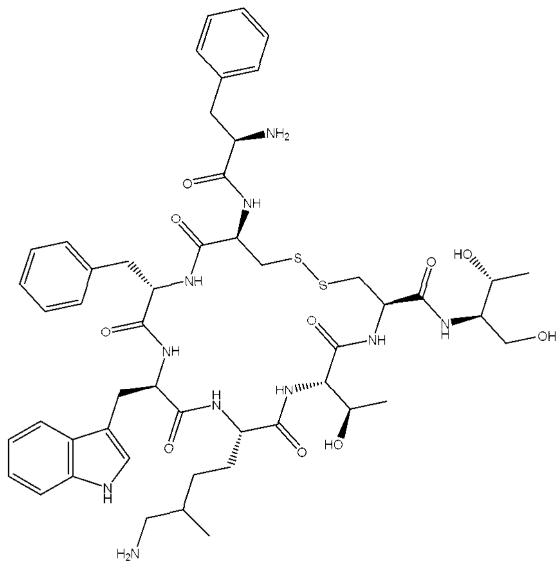

core amino acid sequence Phe-DTrp-Lys(d)-Thr

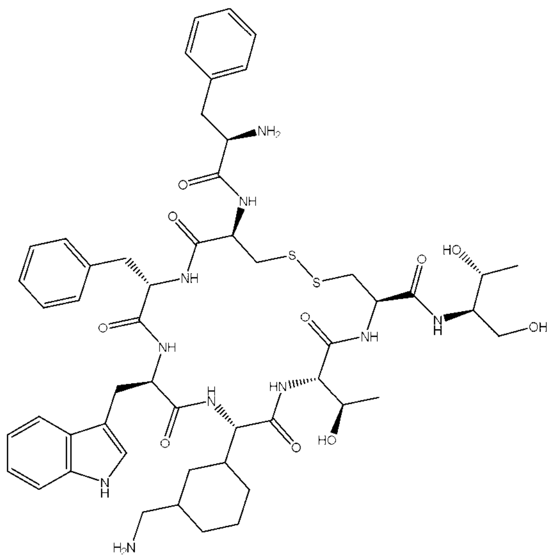

core amino acid sequence Phe-DTrp-Lys(j)-Thr

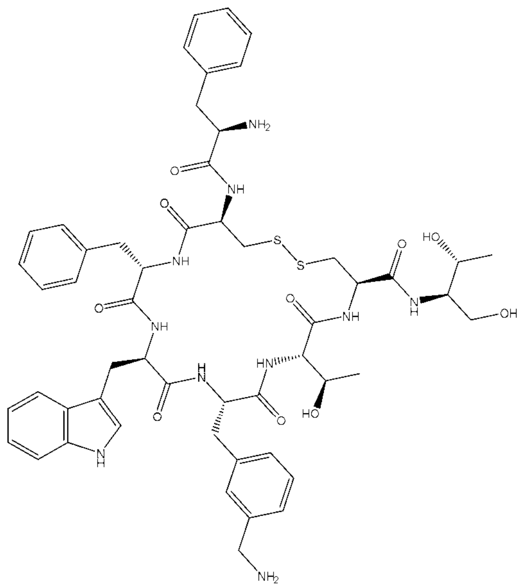

core amino acid sequence Phe-DTrp-Lys(n)-Thr

Another objective of the present invention is to provide a somatostatin analog chelate, wherein the chelate is formed by removing a hydrogen atom from the N-terminal $NH_2$ group of Formula II and coupling it either directly to a chelating group or indirectly via a linker, and is represented by the following structural formula:

chelating group-(linker)-dehydrogenated Formula II

wherein the chelating group includes any suitable chelating group known in the relevant art. Preferably, the chelating group is selected from diethylenetriaminepentaacetic acid (DTPA), 1,4,7,10-tetraazacyclododecane-1,4,7,10-tetraacetic acid (DOTA), 1,4,8,11-tetraazacyclotetradecane-1,4,8,11-tetraacetic acid (TETA), a substituted ethylenediaminetetraacetic acid (EDTA), or a substituted DTPA. More preferably, the chelating group is DTPA or DOTA.

DTPA, DOTA, TETA, and EDTA have the following structure:

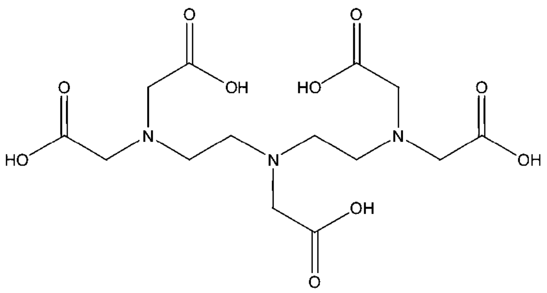

**DTPA**

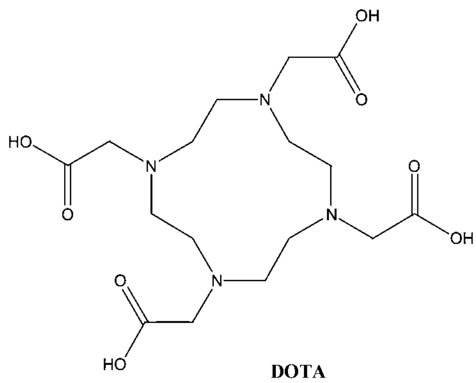

**DOTA**

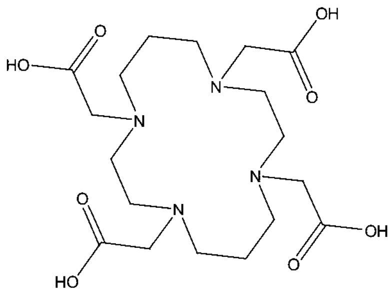

TETA

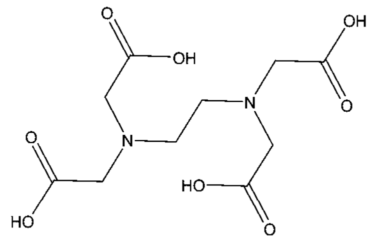

EDTA

Suitable linkers include those disclosed in German Patent GB-A-2,225,579, such as divalent residues of aminocarboxylic acids, including β-alanine (β-Ala) or divalent residues derived from 6-aminocaproic acid, as represented by the following structures:

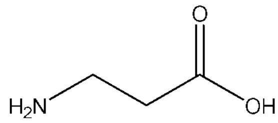

β-Ala

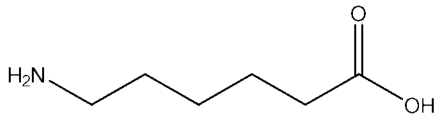

6-aminohexanoic acid

In one embodiment, the somatostatin analog chelate has the following structure:

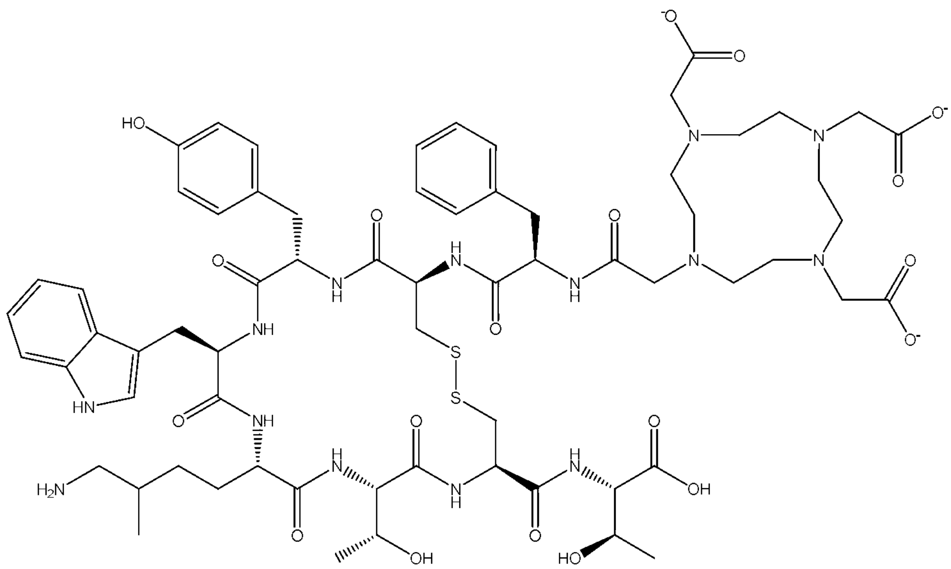

When the chelating group binds a detectable element, the compound is applied for Computed Axial Tomography (CAT) scanning. Suitable detectable elements include, for example, fluorine-19 ($^{19}F$), heavy metal ions, or preferably rare earth metal ions, such as paramagnetic ions (e.g., $Gd^{3+}$, $Fe^{3+}$, $Mn^{2+}$, and $Cr^{2+}$); fluorescent metal ions, such as $Eu^{3+}$; and radionuclides, including radioactive lanthanides, gamma-emitting radionuclides, beta-emitting radionuclides, alpha-emitting radionuclides, Auger electron-emitting radionuclides, and positron-emitting radionuclides, such as gallium-68 ($^{68}Ga$).

Suitable gamma-emitting radionuclides include those useful in diagnostic techniques, such as radionuclides of gallium, indium, technetium, ytterbium, rhenium, terbium, thallium, and samarium, including $^{67}Ga$, $^{111}In$, $^{99m}Tc$, $^{161}Tb$, $^{169}Yb$, and $^{186}Re$.

Suitable beta-emitting radionuclides include those suitable for therapeutic applications, such as yttrium-90 ($^{90}Y$), copper-67 ($^{67}Cu$), copper-64 ($^{64}Cu$), lutetium-177 ($^{177}Lu$), rhenium-186 ($^{186}Re$), rhenium-188 ($^{188}Re$), erbium-169 ($^{169}Er$), tin-121 ($^{121}Sn$), tellurium-127 ($^{127}Te$), praseodymium-143 ($^{143}Pr$), gold-198 ($^{198}Au$), palladium-109 ($^{109}Pd$), dysprosium-165 ($^{165}Dy$), phosphorus-32 ($^{32}P$), praseodymium-142 ($^{142}Pr$), lead-212 ($^{212}Pb$), and samarium-156

($^{156}Sm$).

Suitable alpha-emitting radionuclides include those used for therapeutic applications, such as astatine-211 ($^{211}At$), bismuth-212 ($^{212}Bi$), actinium-225 ($^{225}Ac$), and thallium-201 ($^{201}Tl$).

The compounds of the present invention may exist in the free form or as pharmaceutically acceptable salts.

Another object of the present invention is to provide the use of somatostatin analogs of Formula II in the preparation of anti-tumor drugs, preferably for the treatment of neuroendocrine tumors, more preferably for the treatment of acromegaly and carcinoid syndrome tumors. In certain embodiments, the anti-tumor drug is used for the treatment, prevention, and/or diagnosis of conditions, diseases, and/or pathologies that express somatostatin receptors SSTR1, SSTR2, SSTR3, SSTR4, and/or SSTR5. Such conditions include, but are not limited to, disorders associated with excessive secretion of growth hormone, gastrointestinal diseases, malignant cell proliferative diseases, angiogenesis-related conditions, and vascular disorders, including transplant vascular diseases, restenosis, and vascular occlusion following vascular injury.

The method comprises administering a therapeutically effective amount of a somatostatin analog, a chelate thereof, a pharmaceutically acceptable salt thereof, or a complex thereof with a detectable element to a subject in need of such treatment.

Another object of the present invention is to provide a salt, prodrug, deuterated derivative, solvate, or hydrate of a somatostatin analog of Formula II.

The present invention utilizes octreotide as a reference compound, which has the following structure, with the core amino acid sequence Phe-D-Trp-Lys-Thr:

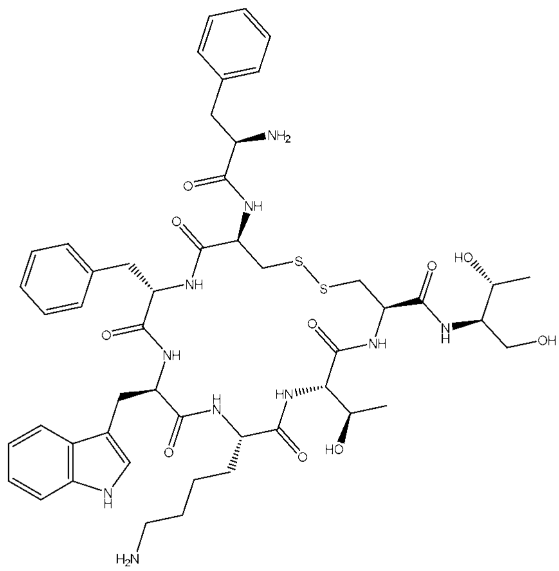

Formula III

The present invention challenges the conventional understanding that the Lys9 residue cannot be replaced without significantly reducing biological activity. The invention modifies the Lys9 residue in somatostatin analogs, resulting in analogs that exhibit higher or at least comparable binding affinity to SSTR2.

**Examples**

Somatostatin Analog Synthesis Methods

The somatostatin analogs of Formula II of the present invention are synthesized using the general synthesis process described below, followed by specific examples of compound synthesis.

Step S1. Swelling of the Resin

Chloro-2-chloro-trityl resin (1 g, degree of substitution: 0.7 mmol per gram) is added to dichloromethane (DCM, 20 mL) and allowed to swell for 30 minutes under nitrogen protection. The DCM is then removed by filtration under vacuum, and the resin is washed three times with N,N-dimethylformamide (DMF).

Step S2. Removal of Fmoc Protecting Group

Fmoc-Thr(OtBu)-OH (2.1 mmol) and diisopropylethylamine (DIPEA, 8.4 mmol) are added to the product of Step S1. After reacting for two hours, the mixture is filtered and washed six times with DMF to obtain Fmoc-Thr(OtBu)-O-2-chloro-trityl resin.

Step S3. Condensation Reaction of Amino Acid

To the product of Step S2, Fmoc-protected amino acid (2.1 mmol), hydroxybenzotriazole (HOBt, 2.1 mmol), 2-(1H-benzotriazol-1-yl)-1H-tetrazole (HBTU, 2.1 mmol), DIPEA (4.2 mmol), and DMF (15 mL) are added. The reaction is allowed to proceed for one hour, then the product is filtered. The obtained Fmoc-protected amino acid-Thr(OtBu)-O-2-chloro-trityl resin is washed three times with DMF (2 minutes each time).

Step S4. Repeat Steps S2 and S3 until all designed polypeptide units are assembled.

Step S5. Resin Shrinkage

In the product obtained from Step S4, the Fmoc protecting group of the last amino acid is removed according to the method of Step S2. The deprotection solution is extracted, and the resin is washed three times with DMF, DCM, and methanol, respectively. The resin is dried under vacuum to obtain the resin polypeptide.

Step S6. Iodine-Methanol Method to Form Disulfide Bonds

The resin polypeptide is dissolved in a mixture of water and acetonitrile, controlling the concentration to approximately 0.001 mol/L. Acetic acid (AcOH) is added to adjust the pH to 4-5. Iodine solution (0.04 mol/L) (approximately 1 g iodine element in 100 mL methanol) is slowly added until the solution no longer turns colorless. The reaction is allowed to proceed for 30 minutes. After the reaction is complete, ascorbic acid (VC) is added to remove excess iodine, and the reaction is allowed to proceed for an additional 30 seconds, until the solution turns colorless. The solution is then lyophilized into a white solid product. The product is separated by preparative high-performance liquid chromatography (prep-HPLC) and lyophilized to obtain the final product. Specifically,

Example 1

Compound 1 (QHJ01-V001)

Lys(a) is a commercially available raw material. The synthesis of Compound 1 is carried out according to the general synthesis process for somatostatin analogs described above, and the specific steps are as follows:

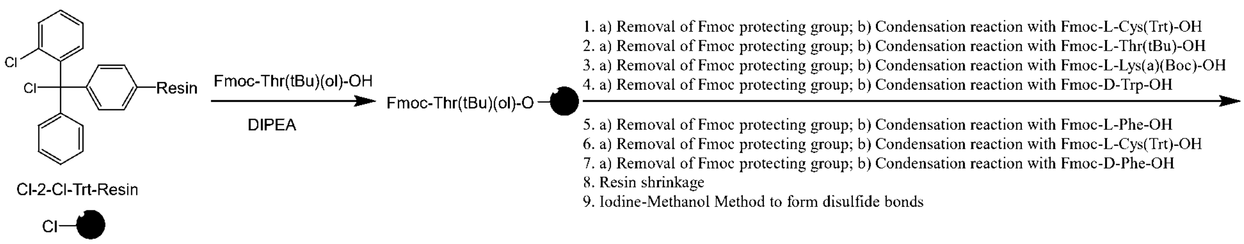

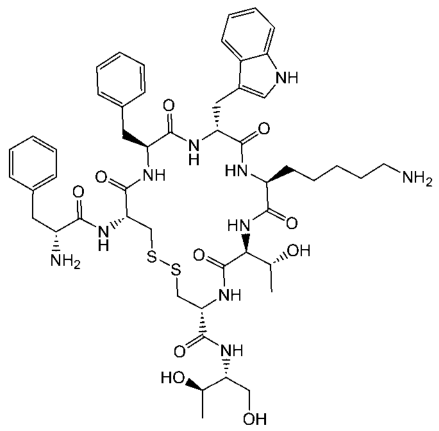

Compound 1

Compound 1 is a white powder with the following properties:
Molecular weight (average): Calculated value 1033.3 g/mol. LC-MS measured value $(M + H)^+$: 1034.4; $(M + 2H)^{2+}$: 517.3.
UPLC measured purity (214 nm): >99%.

Example 2

Compound 2 (QHJ01-V017)

The synthesis steps for the Lys(b) derivative residue in Compound 2 are as follows:

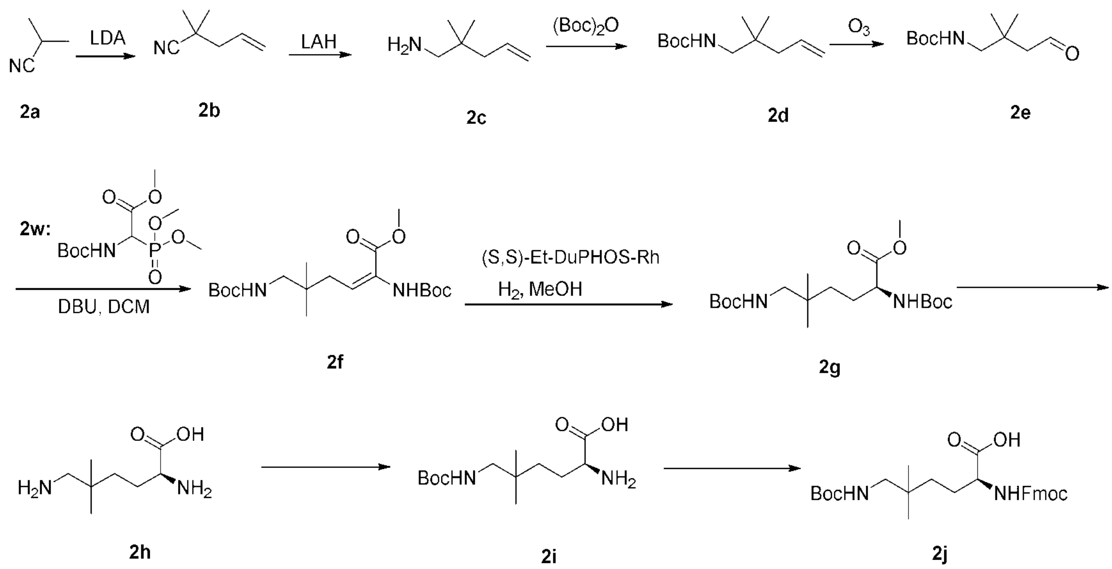


Synthesis of compound 2b is as follows:

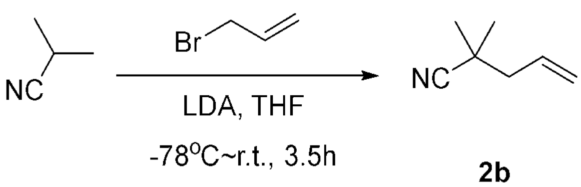


Isobutyronitrile 2a (2.0 g) was dissolved into tetrahydrofuran (THF, 20 mL), and the temperature was lowered to -78°C. Lithium diisopropylamide solution (LDA 15.9 mL, 2 M in THF) was added dropwise, and the mixture was stirred at -78°C for 45 minutes. 3-Bromopropylene (7.1 g) was then added dropwise. The temperature of the reaction solution was gradually raised to room temperature, and the reaction was allowed to proceed at room temperature for 3.5 hours. The reaction solution was quenched with water (50 mL), and the aqueous phase was extracted with n-hexane (3 × 70 mL). The

organic phase was dried over anhydrous sodium sulfate, filtered, and concentrated to yield compound 2b (3.12 g) as a yellow oil.

Compound 2b has the following properties :

$^1$H NMR (400 MHz, DMSO) δ 5.91 - 5.75 (m, 1H), 5.25 - 5.12 (m, 2H), 2.30 - 2.26 (m, 2H), 1.29 (s, 6H).

Synthesis of compound 2c is as follows:

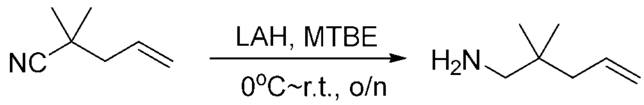


**2b** **2c**

Compound 2b (2.55 g) was dissolved in methyl tert-butyl ether (MTBE, 30 mL). Lithium aluminum hydride (LAH, 3.56 g) was added in batches at 0°C. The mixture was slowly warmed to room temperature and allowed to react overnight at room temperature. The reaction solution was quenched with 6 M sodium hydroxide (NaOH, 20 mL, aqueous) in an ice bath. The aqueous phase was extracted with MTBE (3 × 40 mL), and the organic phase was dried over anhydrous sodium sulfate, filtered, and concentrated to yield compound 2c (2.64 g) as a light yellow viscous oil.

Compound 2c has the following properties:

$^1$H NMR (400 MHz, DMSO) δ 5.88 - 5.71 (m, 1H), 5.07 - 4.95 (m, 2H), 2.28 (s, 2H), 1.94 - 1.89 (m, 2H), 0.77 (s, 6H).

Synthesis of Compound 2d is as follows:

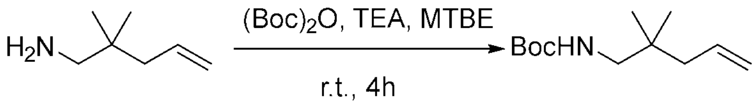


**2c** **2d**

Compound 2c (2.64 g) was dissolved in methyl tert-butyl ether (MTBE, 20 mL). Di-tert-butyl dicarbonate ($(Boc)_2O$, 7.01 g) and triethylamine (TEA, 6 mL) were added, and the mixture was reacted at room temperature for 4 hours. The reaction solution was diluted with water (20 mL), and the aqueous phase was extracted with ethyl acetate (EA, 3 × 40 mL). The organic phase was washed with a saturated aqueous sodium chloride solution (1 × 50 mL), dried over anhydrous sodium sulfate, filtered, and concentrated. The residue obtained by concentration was purified by silica gel column chromatography (petroleum ether/ethyl acetate = 5/1) to yield compound 2d (2.08 g).

Compound 2d has the following properties:

$^1$H NMR (400 MHz, DMSO) δ 6.79 (t, J = 6.1 Hz, 1H), 5.87 - 5.74 (m, 1H), 5.06 - 4.93 (m, 2H), 2.76 (d, J = 6.4 Hz, 2H), 1.88 (d, J = 7.5 Hz, 2H), 1.38 (s, 9H), 0.76 (s, 6H).

Synthesis of Compound 2e is as follows:

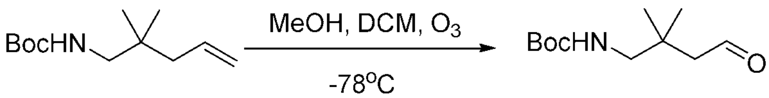


**2d** **2e**

Compound 2d (1.0 g) was dissolved in a mixed solution of methanol (MeOH, 20 mL) and dichloromethane (DCM, 20 mL). After the temperature was lowered to -78°C, ozone ($O_3$) was continuously introduced until the reaction solution turned blue. The reaction solution was then concentrated and dried in a rotary evaporator, and then used directly in the next step.

Compound 2e has the following properties:

$^1$H NMR (400 MHz, DMSO) δ 11.51 (s, 1H), 6.67 (t, J = 6.2 Hz, 1H), 2.79 - 2.75 (m, 2H), 1.46 - 1.41 (m, 2H), 1.38 (s, 9H), 0.82 (s, 6H).

Synthesis of Compound 2f is as follows:

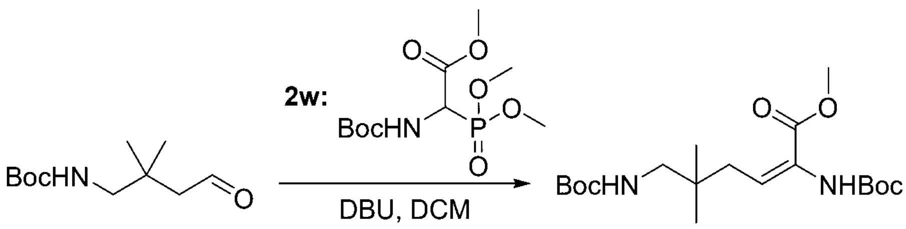


2e 2f

Compound 2e (921.1 mg) was dissolved in dichloromethane (DCM, 10 mL). Compound 2w (1.463 g) and DBU (758 mg) were added, and the mixture was reacted at room temperature overnight. The reaction solution was diluted with water (20 mL), and the aqueous phase was extracted with DCM (3 × 40 mL). The organic phase was washed with water (1 × 50 mL), dried over anhydrous sodium sulfate, filtered, and concentrated. The concentrated residue was purified by column chromatography to yield compound 2f (550 mg).

Compound 2f has the following properties:

$^1$H NMR (400 MHz, DMSO) δ 8.32 (br, 1H), 6.80 (t, J = 6.1 Hz, 1H), 6.35 (br, 1H), 3.66 (s, 3H), 2.77 (d, J = 6.4 Hz, 2H), 2.01 (d, J = 7.8 Hz, 2H), 1.38 (s, 18H), 0.78 (s, 6H).

Synthesis of Compound 2g is as follows:

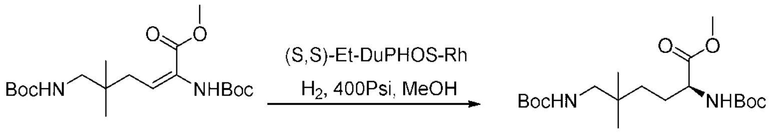


2f 2g

Compound 2f (550 mg) was dissolved in methanol (MeOH, 20 mL). (S,S)-Et-DuPHOS-Rh (13 mg) was added, and the mixture was reacted at room temperature for 3 hours under a hydrogen atmosphere (400 psi). The solid was filtered off, and the filtrate was concentrated to yield compound 2g (630 mg, crude).

Compound 2g has the following properties:

$^1$H NMR (400 MHz, DMSO) δ 7.11 (d, J = 7.5 Hz, 1H), 6.78 (t, J = 6.2 Hz, 1H), 3.87 - 3.79 (m, 1H), 3.61 (s, 3H), 2.77 - 2.68 (m, 2H), 1.64 - 1.44 (m, 2H), 1.41 - 1.34 (m, 18H), 1.24 - 1.19 (m, 1H), 1.12 - 1.02 (m, 1H), 0.74 (s, 6H).

Synthesis of Compound 2h is as follows:

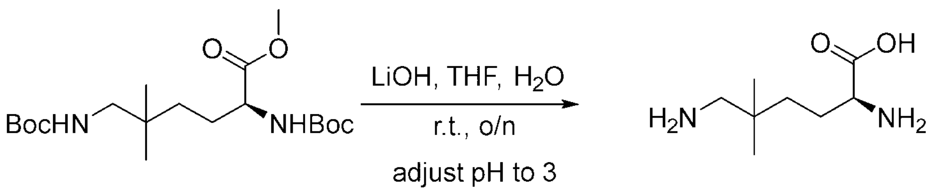


2g 2h

Compound 2g (630 mg, crude) was dissolved in a mixed solution of tetrahydrofuran (THF, 10 mL) and water ($H_2O$, 10 mL), and lithium hydroxide monohydrate ($LiOH{\cdot}H_2O$, 138 mg) was added. The mixture was reacted at room temperature overnight. After the reaction solution was concentrated, the pH of the solution was adjusted to approximately 3. The aqueous phase was extracted with ethyl acetate (EA, 3 × 30 mL), dried over anhydrous sodium sulfate, filtered, and the filtrate was concentrated to yield compound 2h (450 mg).

Synthesis of Compound 2i is as follows:

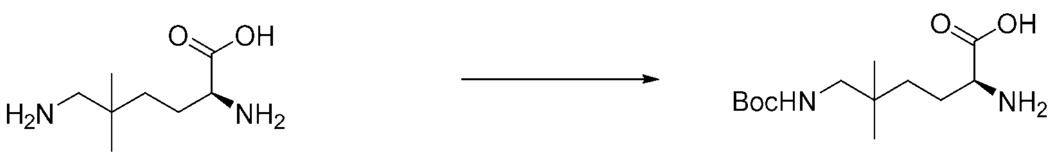

2h 2i

Compound 2h (210 mg) was dissolved in 2 M sodium bicarbonate ($NaHCO_3$, 2.4 mL, 420 mg), and a solution of copper sulfate pentahydrate ($CuSO_4 \cdot 5H_2O$, 300 mg) in water (2.40 mL) was added. Sodium bicarbonate ($NaHCO_3$, 100 mg) was then added, followed by di-tert-butyl dicarbonate ($(Boc)_2O$, 414 mg) dissolved in 3 mL acetone. The mixture was stirred for 24 hours. Methanol (1 mL) was added to the solution, and stirring was continued for an additional 14 hours. Water (2 mL) was added to the mixture, and the product was filtered, washed with water (3 × 2 mL), and dried to yield a light blue solid.

To remove copper, the light blue Cu-chelate was suspended in water (27 mL). 8-Quinolinol (234 mg) was then added, and the mixture was stirred for 18 hours, forming a light salad green precipitate. The suspension was filtered, the precipitate was washed with water (3 × 7 mL), and the filtrate was washed with ethyl acetate (3 × 15 mL). The aqueous layer was lyophilized to yield 120 mg of a white solid.

Compound 2i has the following property:
LC-MS: $[M + H]^*$ = 275.2.

Synthesis of Compound 2j is as follows:

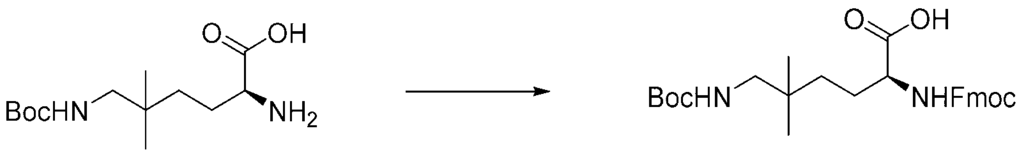

2i 2j

Compound 2i (120 mg) was dissolved in a mixed solution of water ($H_2O$, 5 mL), and a solution of Fmoc-OSu (168 mg) dissolved in acetonitrile (ACN, 5 mL) was added. The mixture was stirred at room temperature for 3 hours. The organic phase was extracted with ethyl acetate (EA, 3 × 6 mL), and the residue was concentrated, and was then purified by flash chromatography (C18 column) to yield compound 2j (150 mg) as a white solid.

Compound 2j has the following properties:

LC-MS: $[M + H]^+$ = 497.4.
$^1H$ NMR (400 MHz, DMSO-$d_6$) δ 12.38 (br, 1H), 7.82 (dd, J = 8.2, 1.3 Hz, 2H), 7.62 (dd, J = 7.5, 1.4 Hz, 2H), 7.50 (td, J = 7.7, 1.2 Hz, 2H), 7.41 (td, J = 7.9, 1.5 Hz, 2H), 6.23 (d, J = 8.4 Hz, 1H), 5.45 - 5.38 (m, 1H), 4.78 (t, J = 5.1 Hz, 1H), 4.42 (d, J = 4.9 Hz, 2H), 4.24 (dt, J = 8.6, 6.2 Hz, 1H), 3.15 - 3.01 (m, 2H), 1.94 - 1.74 (m, 2H), 1.42 (s, 9H), 1.40 - 1.25 (m, 2H), 0.85 (s, 6H).

Synthesis of Compound 2

Compound 2 is synthesized according to the general synthesis process for somatostatin analogs described above. The specific steps for the synthesis are as follows:

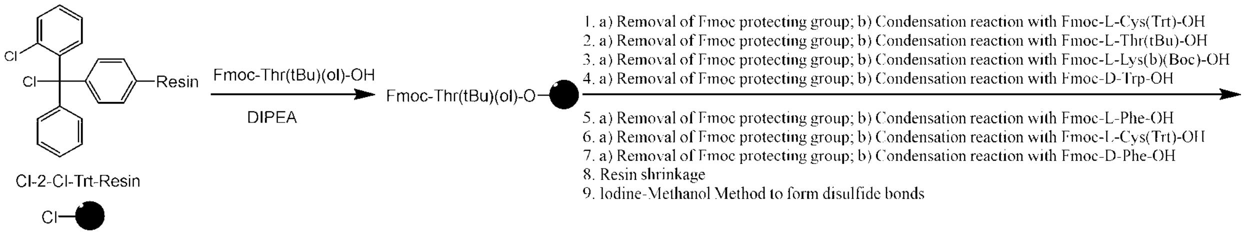


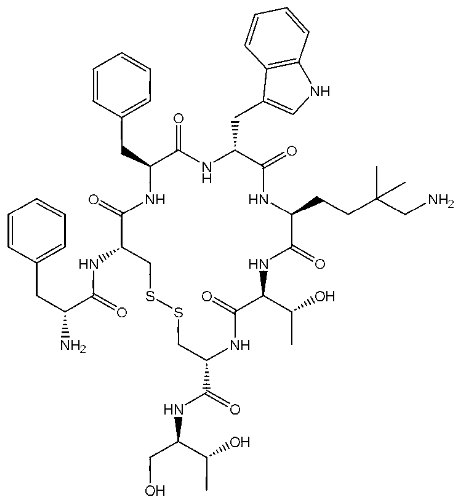

Compound **2**

Compound 2 is a white powder with the following properties:

Molecular weight (average): Calculated value 1047.3 g/mol. LC-MS measured value $(M + 2H)^{2+}$: 524.3. UPLC measured purity (214 nm): >99%.

Example 3

Compound 3 (QHJ01-V002)

The synthesis steps for the Lys(d) derivative residue in Compound 3 are as follows:

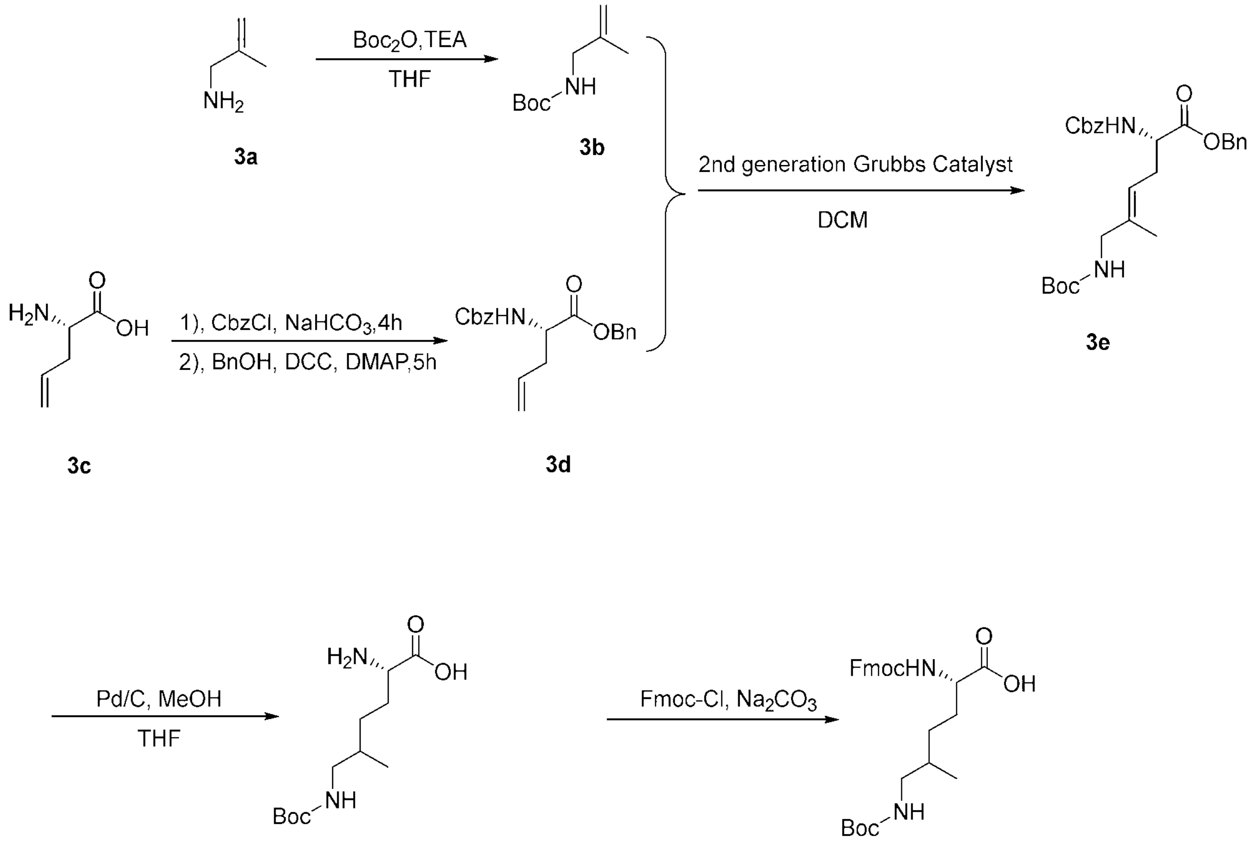


3a 3b 3e 3c 3d 3f 3g

Synthesis of compound 3b is as follows:

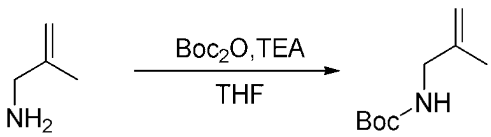


3a 3b

Compound 3a (7.98 g) was dissolved in tetrahydrofuran (THF, 100 mL). Triethylamine (TEA, 11.35 g) and di-tert-butyl dicarbonate ($(Boc)_2O$, 24.50 g) were added, and the reaction mixture was stirred at room temperature overnight. The reaction solution was diluted with water (50 mL), and the aqueous phase was extracted with ethyl acetate (EA, 3 × 50 mL).

After the organic phases were combined, they were dried over anhydrous sodium sulfate, filtered, and concentrated. The concentrated residue was purified by silica gel column chromatography (petroleum ether/ethyl acetate = 9/1) to yield compound 3b (13.4 g).

Synthesis of Compound 3d is as follows:

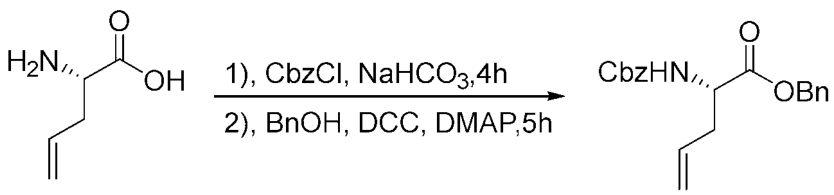


3c 3d

Compound 3c (13.4 g) was dissolved in an aqueous sodium bicarbonate ($NaHCO_3$) solution (29.36 g, 30 mL). Benzyl chloroformate (CbzCl, 29.82 g) was added dropwise at room temperature, and the mixture was reacted at room

temperature overnight. The reaction solution was adjusted to pH 2-3 with 1 M hydrochloric acid (HCl), and the aqueous phase was extracted with ethyl acetate (EA, 3 × 100 mL). The organic phases were combined, dried over anhydrous sodium sulfate, filtered, and concentrated to yield an amide intermediate (40.5 g, crude product). The amide intermediate and benzyl alcohol (12.58 g) were dissolved in dichloromethane (DCM, 100 mL). N,N'-Dicyclohexylcarbodiimide (DCC, 26.40 g) and 4-(dimethylamino)pyridine (DMAP, 1.42 g) were added at 0°C. The reaction mixture was stirred at room temperature for 5 hours. The white solid was filtered off, and the filtrate was concentrated. The residue obtained by concentration was purified by silica gel column chromatography (petroleum ether/ethyl acetate = 7/3) to yield compound 3d (6.7 g).

Synthesis of Compound 3e is as follows:

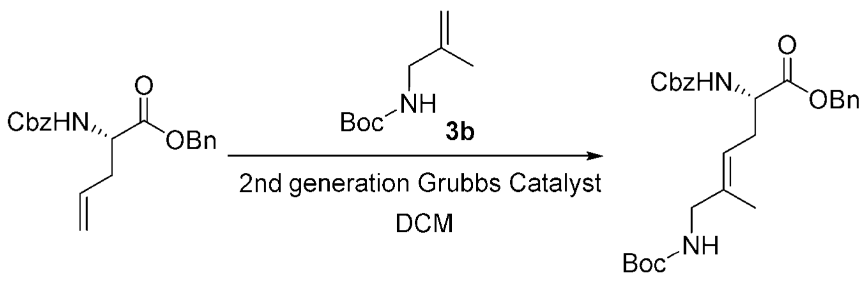


3d

3e

Compound 3d (5.64 g) and compound 3b (8.53 g) were dissolved in dichloromethane (DCM, 50 mL). The second-generation Grubbs catalyst (833 mg) was added, and the mixture was stirred at 60°C overnight. After cooling the reaction solution, water (50 mL) was added to dilute it, and the aqueous phase was extracted with DCM (3 × 50 mL). The organic phases were combined, dried over anhydrous sodium sulfate, filtered, and concentrated. The concentrated residue was purified by silica gel column chromatography (petroleum ether/dichloromethane = 1/1) to yield compound 3e (4.44 g).

Synthesis of Compound 3g is as follows:

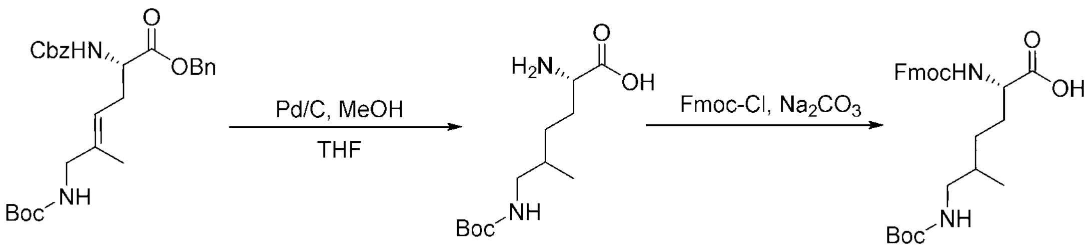


3e

3f

3g

Compound 3e (4.0 g, 8.30 mmol) was dissolved in methanol (30 mL). After nitrogen displacement, 10% palladium on carbon (Pd/C, 500 mg) was added, followed by hydrogen displacement. The mixture was reacted at room temperature overnight under a hydrogen atmosphere. The solid was filtered off, the filtrate was dried, and the obtained residue (compound 3f) was used directly in the next step.

Compound 3f (1.7 g) and sodium carbonate ($Na_2CO_3$, 1.3 g) were dissolved in dioxane (20 mL) and water (6 mL). Fmoc-chloride (Fmoc-Cl, 1.68 g) was added at 0°C. The mixture was stirred at room temperature for 30 minutes and then quenched with water (20 mL). The aqueous phase was extracted with ethyl acetate (EA, 3 × 40 mL). The organic phases were combined, washed with a saturated aqueous sodium chloride solution (1 × 60 mL), dried over anhydrous sodium sulfate, filtered, and concentrated. The concentrated residue was purified by preparative high-performance liquid chromatography (Prep-HPLC) using acetonitrile/water (containing 0.5% formic acid) to yield compound 3g (300 mg).

Compound 3g has the following properties:

Molecular weight (calculated value): 482.6. LC-MS: $[M-H]^-$ = 481.2;

Purity: 97.39%; Rt = 1.814 min.

$^1H$ NMR (400 MHz, DMSO) δ 7.897-7.878 (m, 2H), 7.678-7.661 (m, 2H), 7.429-7.307 (m, 4H), 6.741-6.726 (m, 1H), 6.420-6.408 (m, 1H), 4.256-4.212 (m, 3H), 3.515-3.499 (m, 1H), 2.822-2.626 (m, 2H), 1.598-1.459 (m, 3H), 1.409 (s, 9H), 1.235-1.193 (m, 2H), 0.786-0.744 (m, 3H).

Synthesis of Compound 3

Compound 3 is synthesized according to the general synthesis process for somatostatin analogs described above. The specific steps for the synthesis are as follows:

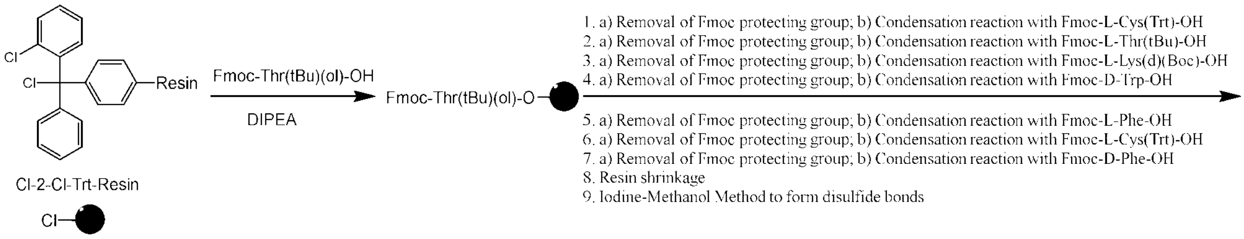


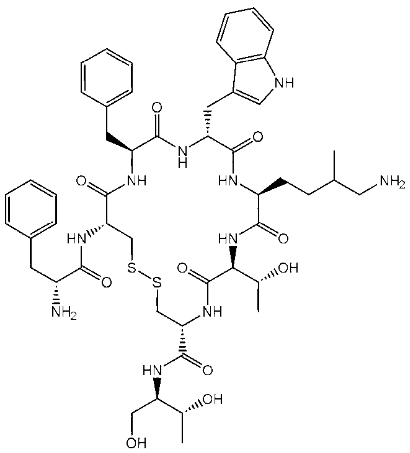

Compound **3**

Compound 3 is a white powder with the following properties:

Molecular weight (average): Calculated value 1033.3 g/mol;
LC-MS measured value $(M + 2H)^{2+}$: 517.4;
UPLC measured purity (214 nm): >99%.

Example 4

Compound 4 (QHJ01-V029)

The synthesis steps for the Lys(j) derivative residue in Compound 4 are as follows:

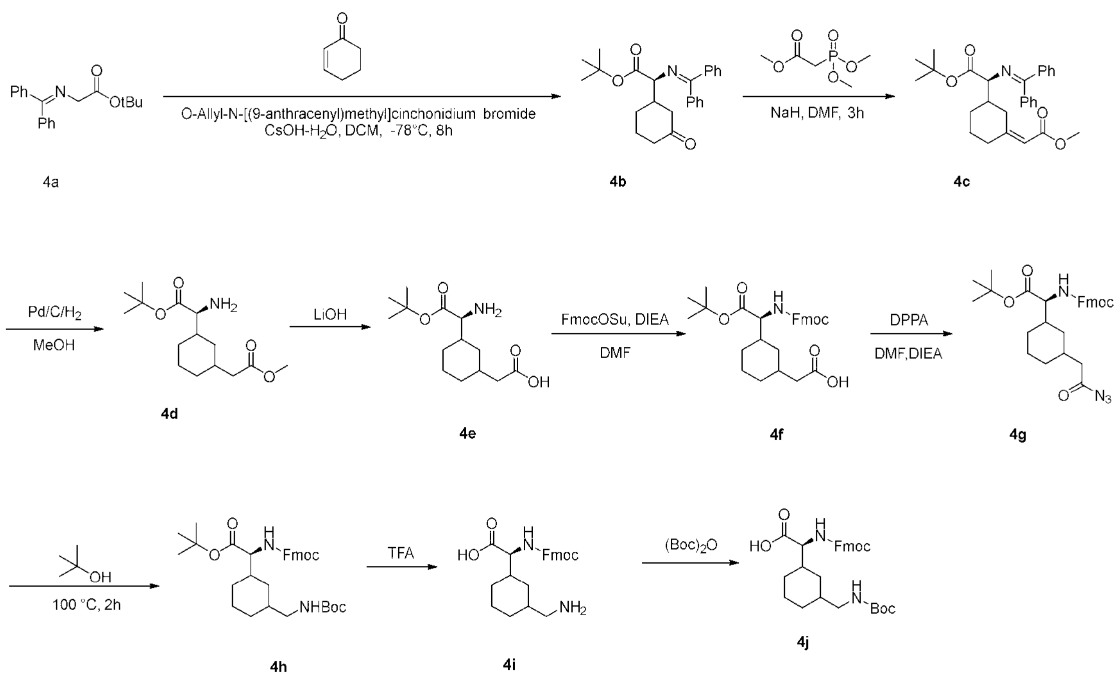


4a 4b 4c 4d 4e 4f 4g 4h 4i 4j

Synthesis of compound 4b is as follows:

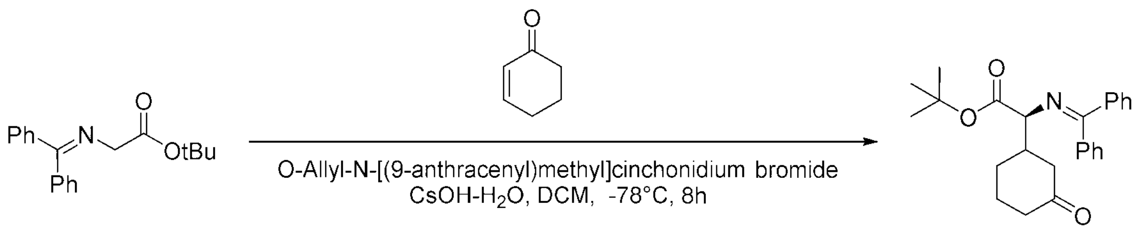


4a **4b**

Compound 4a (10 g) was dissolved in dichloromethane (DCM, 60 mL). O-Allyl-N-[(9-anthracenyl)methyl] cinchonidium bromide (2.05 g) and cesium hydroxide monohydrate ($CsOH \cdot H_2O$, 57 g) were added, and the mixture was cooled to -78°C. A solution of 2-cyclohexene-1-one (10.4 g) in DCM (20 mL) was slowly added dropwise. The reaction mixture was kept at -78°C and stirred for 6 hours.

The reaction solution was poured into water (50 mL) for quenching, and the aqueous phase was extracted with ethyl acetate (EA, 3 × 50 mL). The organic phase was washed with a saturated aqueous sodium chloride solution (1 × 70 mL), dried over anhydrous sodium sulfate, filtered, and concentrated. The concentrated residue was purified by silica gel column chromatography (petroleum ether/ethyl acetate = 10/1) to yield compound 4b (7.2 g) as a white solid.

Compound 4b has the following properties:

LC-MS: $[M + H]^+ = 392.4$.

$^1$H NMR (400 MHz, $CDCl_3$) δ 7.70 - 7.68 (m, 2H), 7.53 - 7.33 (m, 6H), 7.19 - 7.14 (m, 2H), 3.92 (d, J = 4.3 Hz, 1H), 2.70 (t, J = 13.1 Hz, 1H), 2.58 - 2.36 (m, 3H), 2.33 - 2.21 (m, 1H), 2.07 - 2.01 (m, 1H), 1.75 - 1.55 (m, 3H), 1.48 (s, 9H).

Synthesis of Compound 4c is as follows:

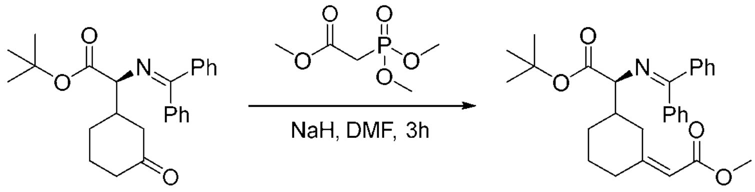


4b 4c

Trimethyl phosphonoacetate (6.7 g) was dissolved into dimethylformamide (DMF, 300 mL) under nitrogen protection. Sodium hydride (NaH, 1.8 g) was added in batches at 0°C. After the mixture was stirred at 0°C for 20 minutes, Compound 4b (7.2 g) was added, and the reaction mixture was stirred at room temperature for 1.5 hours.

The reaction solution was poured into water (500 mL) for quenching, and the aqueous phase was extracted with ethyl acetate (EA, 3 × 500 mL). The organic phase was washed with a saturated aqueous sodium chloride solution (1 × 700 mL), dried over anhydrous sodium sulfate, filtered, and concentrated. The concentrated residue was purified by silica gel column chromatography (petroleum ether/ethyl acetate = 10/1) to yield compound 4c (7.2 g).

Compound 4c has the following property:

LC-MS: $[M + H]^+$ = 448.4.

Synthesis of Compound 4d is as follows:

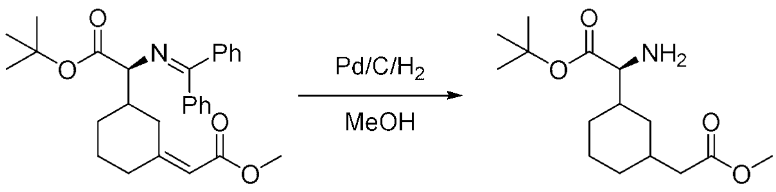


4c 4d

Compound 4c (5 g) was dissolved in methanol (MeOH, 100 mL). Palladium on carbon (Pd/C) was added, and the mixture was stirred at room temperature for 3 hours under a hydrogen atmosphere. The solid was filtered off, and the filtrate was rotary evaporated to obtain compound 4d, which was used directly in the next step of the reaction.

Compound 4d has the following properties:

LC-MS: [M + H]* = 286.3.

Synthesis of Compound 4e is as follows:

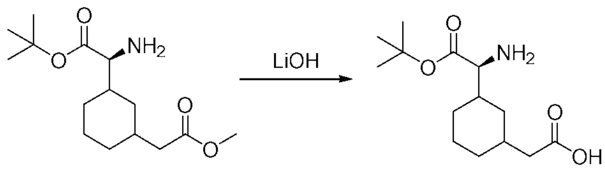


4d 4e

Compound 4d (3.19 g) was dissolved in a mixed solution of methanol (MeOH) and water ($H_2O$, 100 mL). Lithium hydroxide (LiOH, 1.4 g) was added, and the mixture was stirred at room temperature for 2 hours. The reaction solution was concentrated, and the residue obtained by concentration was purified by silica gel column chromatography (dichloromethane/methanol = 5/1) to yield compound 4e (3 g) as a colorless oil.

Compound 4e has the following property:

LC-MS: [M + H]* = 272.2.

Synthesis of Compound 4f is as follows:

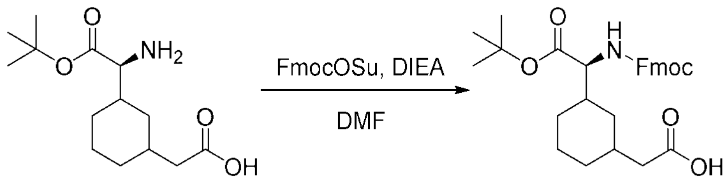


4e 4f

Compound 4e (3 g) was dissolved in DMF (50 mL). FmocOSu (11.2 g) and DIEA (4.3 g) were added, and the mixture was stirred at room temperature for 5 hours. The reaction solution was diluted with water (80 mL), and the aqueous phase was extracted with ethyl acetate (3×100 mL). The organic phase was washed with a saturated aqueous sodium chloride solution (1×150 mL), then dried over anhydrous sodium sulfate, filtered, and concentrated. The residue obtained from the concentration step was purified by silica gel column chromatography (petroleum ether/ethyl acetate = 1/1) to yield compound 4f (3 g).

Compound 4f has the following properties:

LC-MS: [M+H]+ = 494.3.

$^1$H NMR (400 MHz, CDCl3): δ 7.76 (d, J = 7.5 Hz, 2H), 7.61 (d, J = 7.4 Hz, 2H), 7.39 (t, J = 7.4 Hz, 2H), 7.33-7.28 (m, 2H), 5.39-5.33 (m, 1H), 4.42-4.37 (m, 2H), 4.25-4.21 (m, 2H), 2.36 (br, 1H), 2.24-2.22 (m, 1H), 1.98-1.41 (m, 16H), 1.38-1.25 (m, 1H), 1.12-0.78 (m, 2H).

Synthesis of compound 4g is as follows:

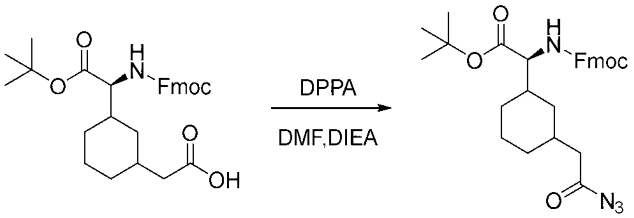


4f 4g

Compound 4f (2.9 g) was dissolved in DMF (20 mL). DPPA (1.94 g) and DIEA (2.3 g) were added, and the mixture was stirred at room temperature for 3 hours. The reaction solution was diluted with water (50 mL), and the aqueous phase was extracted with ethyl acetate (3×70 mL). The organic phase was washed with a saturated aqueous sodium chloride solution (1×100 mL), then dried over anhydrous sodium sulfate, filtered, and concentrated. The residue obtained from the concentration step was purified by silica gel column chromatography (petroleum ether/ethyl acetate = 5/1) to yield compound 4g (1.13 g) as a white solid.

Compound 4g has the following properties:

LC-MS: [M+H]+ = 519.2.

$^1$H NMR (400 MHz, CDCl3): δ 7.77 (d, J = 7.5 Hz, 2H), 7.60 (d, J = 7.3 Hz, 2H), 7.40 (t, J = 7.4 Hz, 2H), 7.32-7.29 (m, 2H), 5.30-5.21 (m, 1H), 4.43-4.40 (m, 2H), 4.23 (t, J = 6.8 Hz, 2H), 2.47-2.13 (m, 2H), 1.95-1.63 (m, 4H), 1.63-1.48 (m, 13H), 1.13-0.78 (m, 3H).

Synthesis of compound 4h is as follows:

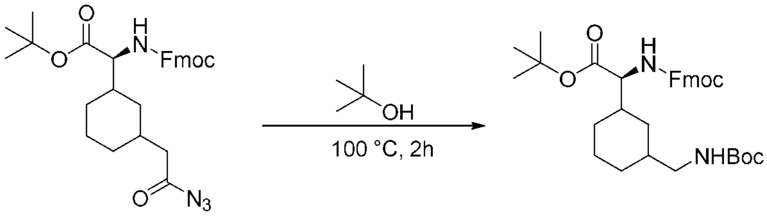


4g 4h

Compound 4g (600 mg) was dissolved in t-BuOH (5 mL), and the mixture was reacted at 100°C for 2 hours under microwave irradiation. The reaction solution was concentrated, and the residue obtained from the concentration step was purified by silica gel column chromatography (petroleum ether/ethyl acetate = 7/1) to yield compound 4h (600 mg).

Compound 4h has the following properties:

LC-MS: [M+H]+ = 565.4.
$^1$H NMR (400 MHz, CDCl3): δ 7.77 (d, J = 7.6 Hz, 2H), 7.61 (d, J = 7.3 Hz, 2H), 7.40 (t, J = 7.4 Hz, 2H), 7.36-7.29 (m, 2H), 5.32-5.29 (m, 1H), 4.40-4.38 (m, 2H), 4.25-4.21 (m, 2H), 3.17-2.88 (m, 2H), 1.84-1.70 (m, 3H), 1.65-1.52 (m, 4H), 1.39 (s, 9H), 1.36 (s, 9H), 1.13-0.67 (m, 3H).

Synthesis of compound 4j is as follows:

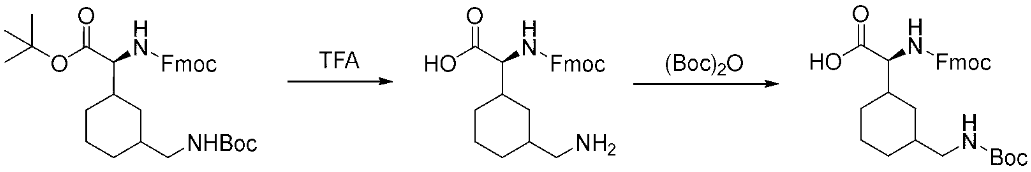


4h 4i 4j

Compound 4h (600 mg) was dissolved in a mixed solution of TFA (10 mL) and DCM (10 mL), and the mixture was reacted at room temperature for 30 minutes. The reaction solution was spin-dried to yield compound 4i, which was directly used in the next step.

Compound 4i (600 mg) was dissolved in DCM (20 mL). (Boc)2O (267 mg) and DIEA (569 mg) were added, and the mixture was reacted at room temperature for 1 hour. The reaction solution was concentrated, and the residue obtained from the concentration step was purified by silica gel column chromatography (dichloromethane/methanol = 10/1) to yield compound 4j (1 g).

Compound 4j has the following properties:

LC-MS: [M+H]+ = 509.4.
$^1$H NMR (400 MHz, DMSO): δ 7.90 (d, J = 7.5 Hz, 2H), 7.76-7.63 (m, 2H), 7.50-7.45 (m, 1H), 7.42 (t, J = 7.4 Hz, 2H), 7.33 (t, J = 7.4 Hz, 2H), 6.81-6.77 (m, 1H), 4.26-4.22 (m, 3H), 3.96-3.80 (m, 1H), 2.90-2.71 (m, 2H), 1.72-1.54 (m, 4H), 1.37 (s, 9H), 1.36-1.30 (m, 2H), 1.15-0.63 (m, 4H).

Synthesis of compound 4 is as follows:

Compound 4 is synthesized according to the general synthesis process for somatostatin analogs described above. The specific steps for the synthesis are as follows:

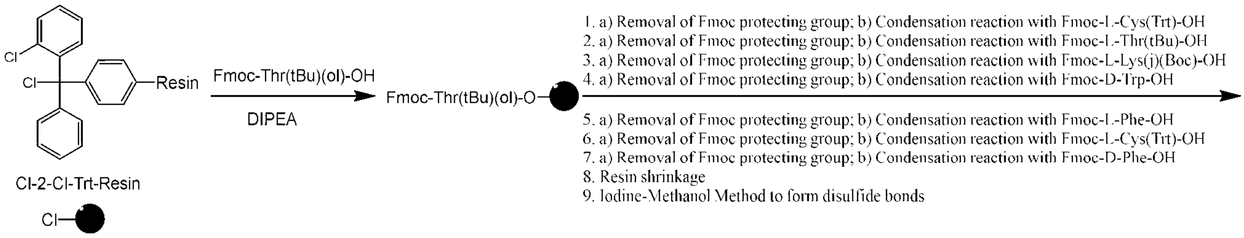


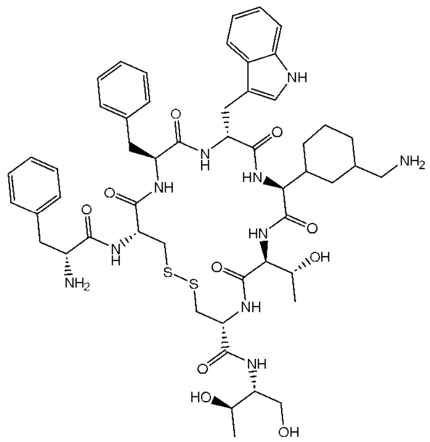

Compound **4**

Compound 4 is a white powder with the following properties:

Average molecular weight: 1059.3 g/mol (calculated value);
The LC-MS measured value is [M + 2H]2+ = 530.4;
The UPLC-measured purity (214 nm) is 93%.

Example 5

Compound 5 (QHJ01-V003)

The Lys(n) residue is a commercially available raw material. Compound 5 is synthesized according to the general synthesis process for somatostatin analogs described above. The specific steps for the synthesis are as follows:

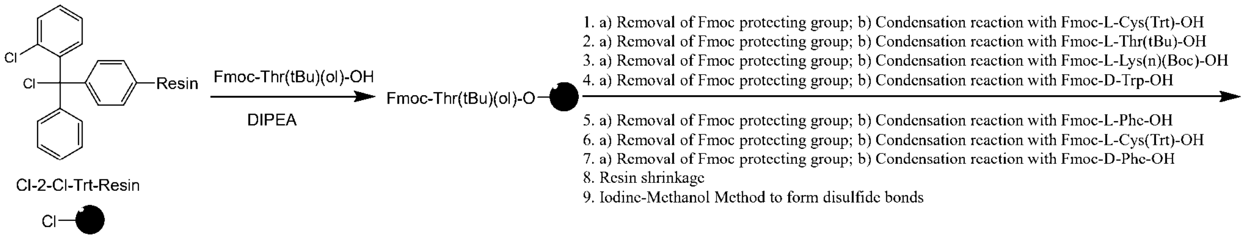


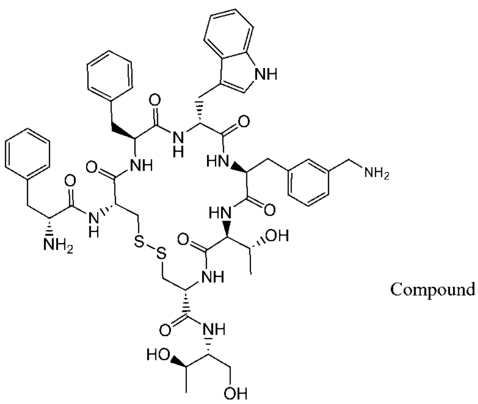

Compound **5**

Compound 5 is a white powder with the following properties:

Average molecular weight of 1067.3 g/mol (calculated value);
LC-MS measured values: $[M + H]^+$ = 1068.4 and [M + 2H]2+ = 534.4; The UPLC-measured purity (214 nm) is 99%.

Example 6

Compound 6 (QHJ01-V059)

Compound 6a is prepared according to the general synthesis process for somatostatin analogs described above.

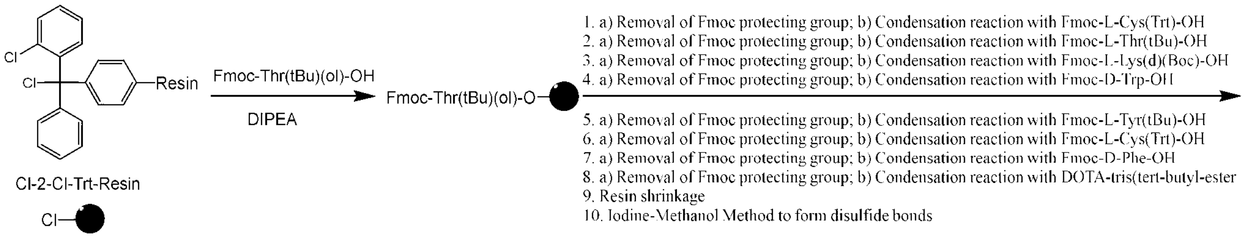


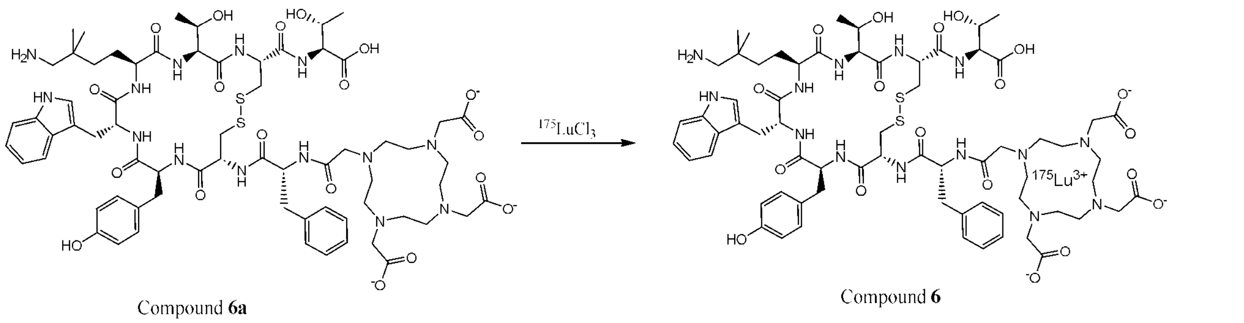


Compound **6a** Compound **6**

Compound 6a is a white powder with the following properties: Chemical formula:

C67H94N14O19S2;
Average molecular weight: 1463.69 g/mol (calculated value);
LC-MS measured values: [M + H]+ = 1463.6 and [M + H]+ = 1485.6; UPLC-measured purity (214 nm) is 99%.

Compound 6 is synthesized according to the following steps :
Compound 6a (5 mg) and 0.1 M sodium acetate (1 mL, pH = 5.5) were mixed, and LuCl3•6H2O (5.6 mg) was slowly added. The mixture was stirred at room temperature for 12 hours, then separated by high-performance liquid chromatography (prep-HPLC) and lyophilized to obtain compound 6.
Compound 6 has the following properties

Chemical formula: C67H91LuN14O19S2;
Molecular weight (average): calculated value 1635.6 g/mol;
LC-MS measured value $[M + H]^+$ = 1635.7 ;
HPLC measured purity (214 nm): >99%.

Example 7

Compound 7p2 (QHJ01-V29p2)

The synthesis steps for the Lys derivative residue in compound 7p2 are as follows:

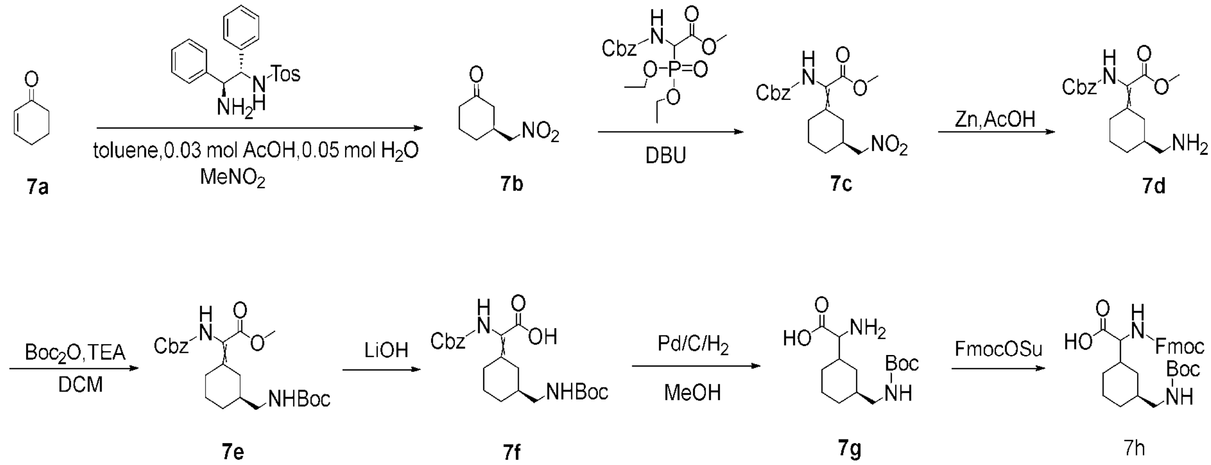

7a 7b 7c 7d
7e 7f 7g 7h

Synthesis of compound 7b is as follows:

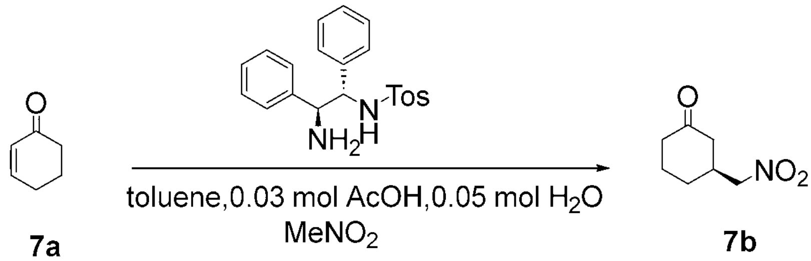

7a 7b

Nitromethane (19.05 g) was dissolved in toluene (70 mL), and (1S,2S)-(+)-N-p-toluenesulfonyl-1,2-diphenylethylenediamine (9.53 g), AcOH (0.45 mL), and $H_2O$ (0.25 mL) were added. After stirring to ensure uniform mixing, a toluene solution (60 mL) of 2-cyclohexene-1-one (25.00 g) was slowly added dropwise. The reaction mixture was kept at room temperature for 8 hours. The reaction solution was poured into water (100.00 mL) to quench the reaction, and the aqueous phase was extracted with ethyl acetate (3×50 mL). The organic phase was washed with a saturated aqueous sodium chloride solution (1×100 mL), then dried over anhydrous sodium sulfate, filtered, and concentrated. The concentrated residue was purified by silica gel column chromatography (petroleum ether/ethyl acetate = 15/1) to yield compound 7b (20.00 g).

Synthesis of compound 7c is as follows:

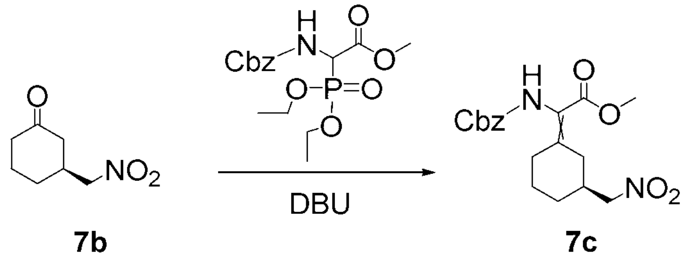

7b 7c

N-CBZ-(diethoxyphosphoryl)glycine methyl ester (54.87 g) was dissolved in DMF (500 mL), and DBU (22.80 mL) was added dropwise at 0°C under nitrogen protection. After the mixture was stirred at 0°C for 20 minutes, Compound 7b (20 g) was slowly added, and the reaction mixture was stirred at room temperature for 5 hours. The reaction solution was poured into water (1000 mL) to quench the reaction, and the aqueous phase was extracted with ethyl acetate (3×500 mL). The organic phase was washed with a saturated aqueous sodium chloride solution (1×700 mL), then dried over anhydrous sodium sulfate, filtered, and concentrated. The concentrated residue was purified by silica gel column chromatography (petroleum ether/ethyl acetate = 10/1) to yield compound 7c (29.3 g).

Synthesis of compound 7d is as follows:

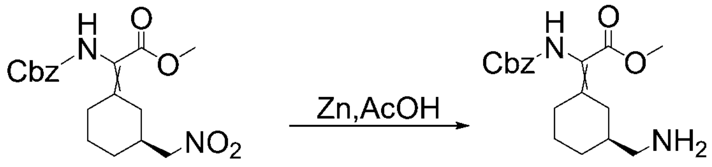


7c 7d

Compound 7c (29.30 g) was placed in AcOH (400 mL) and stirred at 0°C under nitrogen protection. Zinc powder (37.0 g) was then added in batches, and the mixture was stirred for 4 hours. The reaction solution was filtered through diatomaceous earth and evaporated to yield the crude product 7d (17.70 g), which was directly used in the next step without further purification.

Synthesis of compound 7e is as follows:

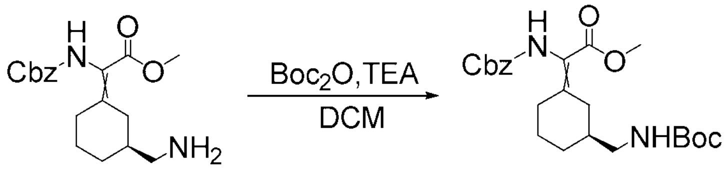


7d 7e

The crude compound 7d (17.70 g) and (Boc)2O (14.65 mL) were dissolved in DCM (50 mL). TEA (8.85 mL) was slowly added, and the mixture was reacted at room temperature for 3 hours. The reaction solution was concentrated, and the residue obtained from the concentration step was purified by silica gel column chromatography (dichloromethane/-methanol = 15/1) to yield compound 7e (8.30 g).

Synthesis of compound 7f is as follows:

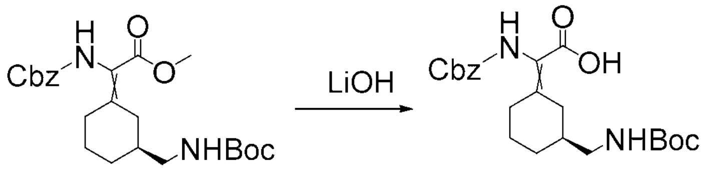


7e 7f

Compound 7e (8.30 g) was dissolved in a mixed solution of MeOH and H2O (100 mL). LiOH (1.61 g) was added, and the mixture was stirred at room temperature for 4 hours. The reaction solution was concentrated, and the residue obtained from the concentration step was purified by silica gel column chromatography (dichloromethane/methanol = 10/1) to yield compound 7f (4.70 g).

Synthesis of compound 7g is as follows:

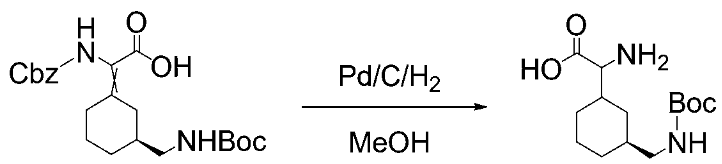


7f 7g

Compound 7f (4.70 g) was dissolved in MeOH (70 mL), and Pd/C was added. The mixture was stirred at room temperature for 6 hours under a hydrogen atmosphere. The solid was filtered off, and the filtrate was dried to yield the crude compound 7g (3.00 g), which was directly used in the next step of the reaction.

Synthesis of compound 7h is as follows:

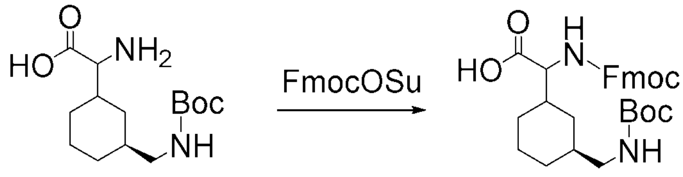


**7g** **7h**

The crude compound 7g (3.00 g) was dissolved in DMF (50 mL), and FmocOSu (3.89 g) and DIEA (2.1 mL) were added. The mixture was stirred at room temperature for 5 hours. The reaction solution was diluted with water (80 mL), and the aqueous phase was extracted with ethyl acetate (3×100 mL). The organic phase was washed with a saturated aqueous sodium chloride solution (1×150 mL), then dried over anhydrous sodium sulfate, filtered, and concentrated. The residue obtained from the concentration step was purified by silica gel column chromatography (dichloromethane/methanol = 10/1) to yield compound 7h (0.65 g).

Compound 7h has the following properties:

LC-MS: [M+H]+ = 509.4;
$^1$H NMR (400 MHz, DMSO-d6): δ 7.89 (d, J = 7.5 Hz, 2H), 7.75 (d, J = 7.5 Hz, 2H), 7.42 (t, J = 7.4 Hz, 2H), 7.33 (t, J = 7.4 Hz, 2H), 6.79 (d, J = 6.0 Hz, 1H), 4.24 (dd, J = 8.2, 4.4 Hz, 3H), 3.85 (s, 1H), 2.84-2.65 (m, 2H), 1.82-1.52 (m, 5H), 1.37 (s, 9H), 1.25-0.91 (m, 1H), 0.74 (dt, J = 22.9, 11.9 Hz, 2H).

Chiral separation of compound 7h is as follows:
Compound 7h was subjected to the first chiral separation using liquid chromatography to obtain 7h-p1+p2 and 7h-p3+p4. Then, 7h-p1+p2 and 7h-p3+p4 were each subjected to a second chiral separation. The chiral separation method is as follows:

Table 1. Chromatographic Method for Separation of 7h into 7h-p1+p2 and 7h-p3+p4

| | |
|---|---|
| Chromatographic column | CHIRALPAK IC-3(IC30CE-NJ008) |
| Column specification | 0.46 cm I.D. x 25 cm L |
| Injection volume | 100 uL |
| Mobile phase | Hexane/EtOH/TFA = 85/15/0.1(V/V/V) |
| Flow rate | 1.0 mL/min |
| Detection wavelength | UV 254 nm |
| Column temperature | 35°C |
| Instrument model | Shimadzu LC-20AT |

The chromatogram of the separation of 7h into 7h-p1+p2 and 7h-p3+p4 is shown in FIG. 9. The retention time of 7h-p1+p2 is 7.826 minutes, and the retention time of 7h-p3+p4 is 9.492 minutes.

Table 2. Chromatographic Method for Separation of 7h-p1+p2 into 7h-p1 and 7h-p2

| | |
|---|---|
| Chromatographic column | CHIRALPAK IE-3 (IE30CE-NJ008) |
| Column specification | 0.46 cm I.D. x 25 cm L |
| Injection volume | 50 uL |
| Mobile phase | Hexane/EtOH/TFA = 80/20/0.1 (V/V/V) |
| Flow rate | 1.0 mL/min |
| Detection wavelength | UV 254 nm |
| Column temperature | 35°C |
| Instrument model | Shimadzu LC-20AT |

The chromatogram of the separation of 7h-peak1+2 into 7h-p1 and 7h-p2 is shown in FIG. 10. The retention time of 7h-p1 is 7.666 minutes, and the retention time of 7h-p2 is 8.420 minutes. After separation, 42 mg of 7h-p1 and 65 mg of 7h-p2 were obtained.

The specific steps for the synthesis of compound 7p2 are:

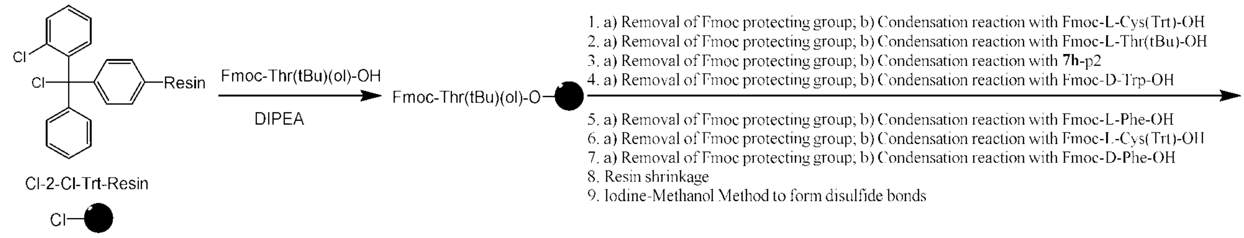


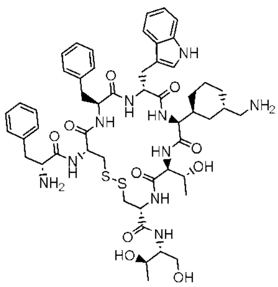
or
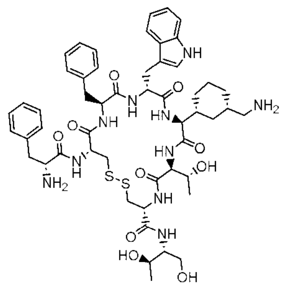
or
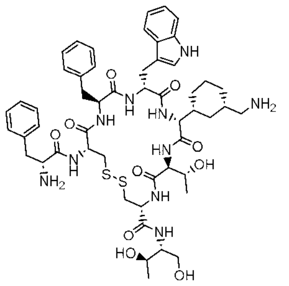
or
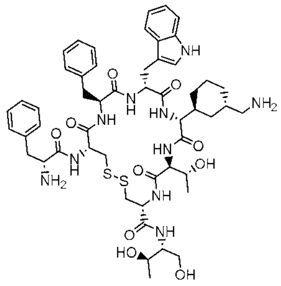

Compound **7p2**

Compound 7p2 is prepared according to the general synthesis process for somatostatin analogs described above.

Compound 7p2 is a white powder with the following properties:

Average molecular weight of 1059.3 g/mol (calculated value);
LC-MS measured value: [M + 2H]2+ = 530.3;
UPLC-measured purity (214 nm) is 96%.

Somatostatin Analog Binding Affinity Measurement

A competitive radioligand binding assay is utilized to measure the binding affinity of the somatostatin analogs of Formula II of the present invention. SSTR2-overexpressing CHO-K1 cells (Hunter Express SSTR2 CHO-K1; DiscoverX) are used in the radioligand competitive binding assay, with $^{125}$I-Tyr$^{11}$-Somatostatin-14 (PerkinElmer) as the radioligand. The assay is performed as follows:

Step S1. Extraction of Cell Membrane

SSTR2-overexpressing CHO-K1 cells (from Discover X) were cultured in complete medium (F12 basal medium with 10% FBS and 1% P/S [100 U/mL penicillin and 100 μg/mL streptomycin]) in a 37°C incubator with 5% CO2. When the cell confluency reached 80-90%, the cells were washed twice with pre-cooled PBS. The cells were then scraped off with a cell scraper and transferred to a 50 mL centrifuge tube, followed by centrifugation at 500×g and 4°C for 10 minutes. The supernatant was discarded, and the cell pellet was retained. Cell membrane lysis buffer (containing 25 mM HEPES, 1 mM EDTA, 2 mM MgCl2, and 1× protease inhibitor, pH = 7.4) was added at a ratio of $4 \times 10^7$ cells/10 mL and placed on ice for 10 minutes. The cells were then disrupted using an IKA disruptor. The operating procedure was 15,000 rpm for 10 seconds, repeated twice. The disrupted cell suspension was centrifuged at 4°C and 650×g for 5 minutes. The supernatant was transferred to a new centrifuge tube and centrifuged at 13,000 rpm and 4°C for 30 minutes. The supernatant was discarded, and the cell membrane pellet was resuspended in 1.6 mL of resuspension buffer, which was cell membrane lysis buffer supplemented with 10% sucrose. The cell membrane lysis buffer was repeatedly aspirated 4 times with a 27-gauge needle to break the DNA. The cell membrane extract was measured for protein concentration using the BCA method and aliquoted and stored at -80°C.

Step S2. Binding to Cell Membrane

(1) Dilution of Compound: The assay buffer used contains 50 mM Hepes, 5 mM MgCl2, 1 mM CaCl2, and 0.5% BSA, pH = 7.4, and the buffer is filtered. The assay buffer is kept at 4°C. The ligand solution is serially diluted using an Agilent Bravo. Each test compound is diluted 3×8 times in succession. The eight binding concentrations of typical

compounds are: 50 nM, 12.5 nM, 3.13 nM, 0.78 nM, 0.20 nM, 0.05 nM, 12.2 pM, and 3.05 pM. The affinity of each concentration is measured twice. Transfer 1 μL of each diluted compound to the corresponding wells of a 96-well V-bottom plate.

(2) Preparation of 125I-SST: The stock solution of 125I-SST was diluted to 3 nM (10×) with binding buffer (containing 50 mM Hepes, 5 mM MgCl2, 1 mM CaCl2, and 0.5% BSA, pH = 7.4, stored at 4°C). Transfer 10 μL of the solution to the corresponding compound well.

(3) Binding Experiment: The extracted cell membrane was diluted to 2.5 μg/90 μL with binding buffer and added to the corresponding wells. The mixture was incubated at 500 rpm on a 25°C shaker for 2 hours. The membrane complex was collected into a 0.5% PEI pre-coated GF/B plate using a Cell Harvester, and rinsed 3 times per well with 200 μL of pre-cooled buffer (4°C) (the same buffer used to dilute the compound). The plate was dried at 37°C for 2 hours. After adding 50 μL of liquid scintillation fluid (MicroScint20, Perkin Elmer) to each well and incubating for 30 minutes, the radioactivity was measured using a Topcount plate reader for 30 seconds per well.

Step S3. Curve Fitting:

For each compound, its 8-point reading data (2× of 8 readings) were imported into XLfit software and fitted to the sigmoidal curve. The EC50 of the compound for SSTR2 was calculated based on the mathematically optimal sigmoidal dose-response model equation:

$$y - A + \frac{(B-A)}{1+\left(\frac{C}{x}\right)^{D}}$$

where A represents the bottom of the curve, B represents the top of the curve, C represents the relative EC50, D represents the Hill slope, y represents the percentage effect value (% Effect) of the curve fitting, and x represents the molar concentration. Accordingly,

$$x = \frac{C}{\left(\frac{(B-A)}{(y-A)} - 1\right)^{1/D}}$$

where A, B, C, and D have the same meanings as defined above, y represents the percentage effect (% Effect) reading, and x represents the molar concentration calculated by curve fitting. When y is 50%, the calculated x corresponds to the absolute EC50 value, as shown in Table 3 and Figures 1 to 8.

Table 3. Binding Affinity of Target Compounds to SSTR2.

| Compound | Code | Relative EC50 M | Absolute EC50 M | Bottom % | Top % | Hill slope |
|---|---|---|---|---|---|---|
| Reference | Octreotide | 6.0E-10 | 3.9E-10 | 17.9 | 99.2 | 0.97 |
| Compound 1 | QHJ01-V001 | 1.3E-09 | 7.4E-10 | 20.6 | 99.3 | 0.92 |
| Compound 2 | QHJ01-V017 | 1.2E-09 | 1.0E-09 | 11.5 | 98.7 | 1.22 |
| Compound 3 | QHJ01-V002 | 2.9E-10 | 2.5E-10 | 9.7 | 98.0 | 1.15 |
| Compound 4 | QHJ01-V029 | 9.1E-11 | 7.1E-11 | 11.7 | 99.9 | 1.07 |
| Compound 5 | QHJ01-V003 | 5.8E-08 | 4.6E-08 | 8.7 | 100.0 | 0.84 |
| Compound 6 | QHJ01-V059 | 1.1E-09 | 8.1E-10 | 13.2 | 98.3 | 0.88 |
| Compound 7p2 | QHJ01-V29p2 | 5.6E-11 | 4.5E-11 | 10.8 | 99.5 | 1.08 |

Conclusion

Modifying the Lys9 group by appropriately increasing spatial tightness and limiting the number of possible side chain conformations enhances the binding affinity of the drug molecule to SSTR2. These innovative modifications challenge the conventional understanding that the replacement of the Lys9 residue inevitably results in a significant reduction in biological activity.

**Brief** Description of the Drawings

FIG. 1 is a graph illustrating the binding affinity of SSTR2 as a function of the concentration of the reference compound, Octreotide.
FIG. 2 is a graph illustrating the binding affinity of SSTR2 as a function of the concentration of Target Compound 1 (QHJ01-V001).
FIG. 3 is a graph illustrating the binding affinity of SSTR2 as a function of the concentration of Target Compound 2 (QHJ01-V017).
FIG. 4 is a graph illustrating the binding affinity of SSTR2 as a function of the concentration of Target Compound 3 (QHJ01-V002).
FIG. 5 is a graph illustrating the binding affinity of SSTR2 as a function of the concentration of Target Compound 4 (QHJ01-V029).
FIG. 6 is a graph illustrating the binding affinity of SSTR2 as a function of the concentration of Target Compound 5 (QHJ01-V003).
FIG. 7 is a graph illustrating the binding affinity of SSTR2 as a function of the concentration of Target Compound 6 (QHJ01-V059).
FIG. 8 is a graph illustrating the binding affinity of SSTR2 as a function of the concentration of Target Compound 7p2 (QHJ01-V29p2).
FIG. 9 is a chromatogram showing the separation of Compound 7h into 7h-p1+p2 and 7h-p3+p4.
FIG. 10 is a chromatogram showing the separation of Compound 7h-p1+p2 into 7h-p1 and 7h-p2.

## Claims

**1.** A somatostatin analog or a pharmaceutically acceptable salt, complex, or chelate thereof, wherein the somatostatin analog comprises:

(a) a cyclic or non-cyclic hexapeptide unit having a residue sequence at positions 2 to 5 of -$X_1$-(D/L-)Trp-$X_2$-$X_3$-; or
(b) a cyclic or non-cyclic octapeptide unit having a residue sequence at positions 2 to 7 of -Cys-$X_1$-(D/L-)Trp-$X_2$-$X_3$-Cys-;

wherein $X_1$ is an $\alpha$-amino acid residue comprising an aromatic group on the C$\alpha$ side chain; $X_2$ is a Lys derivative; and $X_3$ is an $\alpha$-amino acid residue, wherein the structure of $X_2$ is represented as follows:

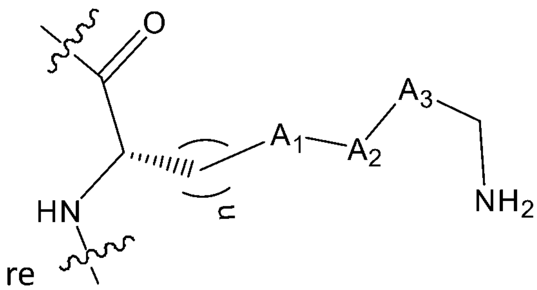

wherein n is 0 or 1;
$A_1$, $A_2$, and $A_3$ are independently selected from $C(R)_2$, O, S, NR, C=O, C=S, C=NR, and C=$C(R)_2$, wherein R is selected from $R_1$, -R'O$R_1$, -R'S$R_1$, -R'N$(R_1)_2$, -R'C(O)$R_1$, -R'S(O)$R_1$, -R'S$(O)_2R_1$, - R'C(O)N$(R_1)_2$, -R'N$(R_1)$C(O)$R_1$, -R'C(O)O$R_1$, and -R'OC(O)$R_1$ ;
$A_2$ may form a double bond or a triple bond with either $A_1$ or $A_3$;
R in $C(R)_2$ forms a 3-8 membered carbon ring with R in its own $C(R)_2$, or with R in the adjacent $C(R)_2$, or with R in a $C(R)_2$ separated by one $C(R)_2$, or forms a 3-8 membered heterocyclic ring with 1-3 heteroatoms selected from N, O, and S;
R' is a connecting bond, or is selected from $C_1$-$C_6$ alkylene, $C_2$-$C_6$ alkenylene, $C_2$-$C_6$ alkynylene, $C_3$-$C_8$ cycloalkylene, $C_3$-$C_8$ cycloalkenylene, halogenated $C_1$-$C_6$ alkylene, halogenated $C_2$-$C_6$ alkenylene, halogenated $C_2$-$C_6$ alkynylene, halogenated $C_3$-$C_8$ cycloalkylene, halogenated $C_3$-$C_8$ cycloalkenylene, $(C_6$-$C_8)$arylene,

3-8 membered heterocyclylene with 1-3 heteroatoms selected from N, O, S, 3-8 membered heteroarylene with 1-3 heteroatoms selected from N, O, and S;

$R_1$ is selected from H, halogen, CN, $NO_2$, $C_1$-$C_6$ alkyl, $C_2$-$C_6$ alkenyl, $C_2$-$C_6$ alkynyl, $C_3$-$C_8$ cycloalkyl, $C_3$-$C_8$ cycloalkenyl, halogenated $C_1$-$C_6$ alkyl, halogenated $C_2$-$C_6$ alkenyl, halogenated $C_2$-$C_6$ alkynyl, halogenated $C_3$-$C_8$ cycloalkyl, halogenated $C_3$-$C_8$ cycloalkenyl, ($C_6$-$C_8$)aryl, 3-8 membered heterocyclic group with 1 to 3 heteroatoms selected from N, O, S, and 3-8 membered heteroaryl with 1 to 3 heteroatoms selected from N, O, and S;

When n is 0, R in $C(R)_2$ at positions $A_1$, $A_2$, and $A_3$ are not H simultaneously.

2. The somatostatin analog of claim 1, wherein the somatostatin analog comprises:

(a) a cyclic or non-cyclic hexapeptide unit having a residue sequence at positions 1 to 6 of Xa-$X_1$-(D/L-)Trp-$X_2$-$X_3$-Xb; or

(b) a cyclic or non-cyclic octapeptide unit having a residue sequence at positions 1 to 8 of $X_o$-Cys-$X_1$-(D/L-)Trp-$X_2$-$X_3$-Cys-Xb;

wherein Xa and Xb are amino acid residues, and $X_o$ is an $\alpha$-amino acid residue comprising an aromatic group on the C$\alpha$ side chain.

3. The somatostatin analog of claim 1 or 2, wherein $X_1$ is selected from the group consisting of Phe, Tyr, Trp, and Phg residues, and $X_3$ is selected from the group consisting of Thr, Ser, Cys, and tyrosylbenzyl residues.

4. The somatostatin analog of claim 1, wherein the somatostatin analog comprises a cyclic octapeptide unit having the following structure:

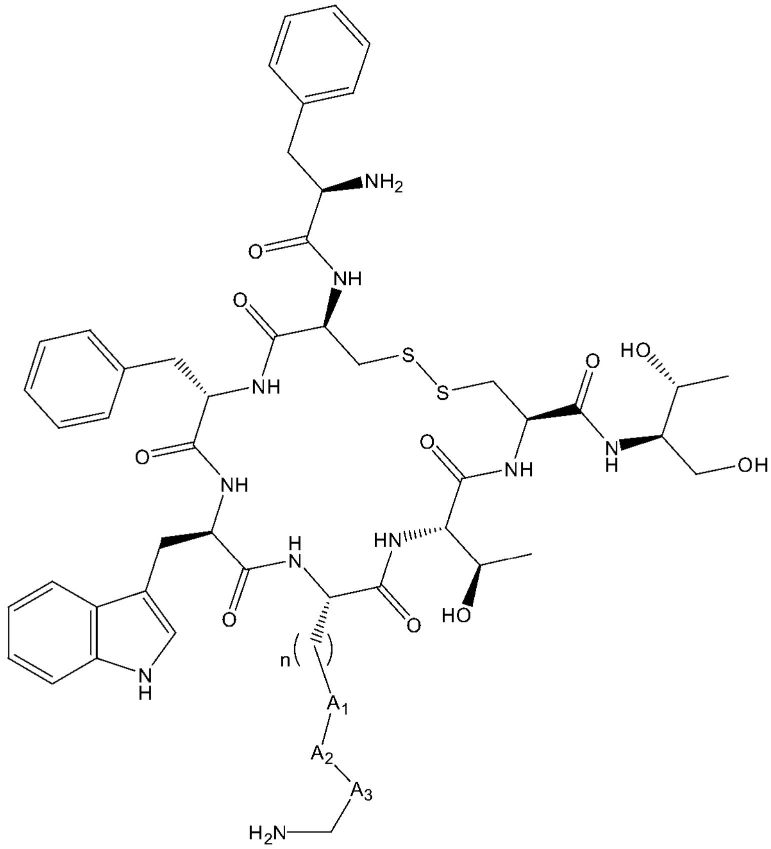

Formula II

5. The somatostatin analog of claim 1 or 4, wherein R in $C(R)_2$ at position $A_1$ and R in $C(R)_2$ at position $A_3$ are connected to form a ring, such that $A_1$, $A_2$, and $A_3$ together form a 3- to 8-membered carbocyclic or heterocyclic ring, wherein the heterocyclic ring comprises 1 to 3 heteroatoms selected from N, O, and S.

Preferably, R in $C(R)_2$ at position $A_1$ and R in $C(R)_2$ at position $A_3$ are connected to form a ring, such that $A_1$, $A_2$, and $A_3$ together form a 5- to 7-membered carbocyclic or heterocyclic ring, wherein the heterocyclic ring comprises 1 to 2 heteroatoms selected from N, O, and S.

**6.** The somatostatin analog of claim 1 or 4, wherein R in $C(R)_2$ at position $A_2$ is connected to R in $C(R)_2$ at position $A_1$ or $A_3$ to form a double bond.

**7.** The somatostatin analog of claim 1 or 4, wherein R' is a connecting bond, or is selected from $C_1$-$C_4$ alkylene, $C_2$-$C_4$ alkenylene, $C_2$-$C_4$ alkynylene, $C_3$-$C_7$ cycloalkylene, $C_3$-$C_7$ cycloalkenylene, halogenated $C_1$-$C_4$ alkylene, halogenated $C_2$-$C_4$ alkenylene, halogenated $C_2$-$C_4$ alkynylene, halogenated $C_3$-$C_7$ cycloalkylene, halogenated $C_3$-$C_7$ cycloalkenylene, ($C_6$-$C_8$)arylene, 3-7 membered heterocyclic radical having 1-3 heteroatoms selected from N, O, and S, or 3-7 membered heteroarylene with 1-3 heteroatoms selected from N, O, and S; and

Wherein $R_1$ is selected from H, halogen, CN, $NO_2$, $C_1$-$C_4$ alkyl, $C_2$-$C_4$ alkenyl, $C_2$-$C_4$ alkynyl, $C_3$-$C_7$ cycloalkyl, $C_3$-$C_7$ cycloalkenyl, halogenated $C_1$-$C_4$ alkyl, halogenated $C_2$-$C_4$ alkenyl, halogenated $C_2$-$C_4$ alkynyl, halogenated $C_3$-$C_7$ cycloalkyl, halogenated $C_3$-$C_7$ cycloalkenyl, ($C_6$-$C_8$)aryl, 3-7 membered heterocyclic group with 1-3 heteroatoms selected from N, O, and S, or 3-7 membered heteroaryl with 1-3 heteroatoms selected from N, O, and S;

Preferrably, wherein R' is a connecting bond, or is selected from $C_1$-$C_3$ alkylene, $C_2$-$C_3$ alkenylene, $C_2$-$C_3$ alkynylene, C5-C6 cycloalkylene, C5-C6 cycloalkenylene, halogenated $C_1$-$C_3$ alkylene, halogenated $C_2$-$C_3$ alkenylene, halogenated $C_2$-$C_3$ alkynylene, halogenated C5-C6 cycloalkylene, halogenated C5-C6 cycloalkenylene, ($C_6$-$C_8$)arylene, 5-6-membered heterocyclylene with 1-2 heteroatoms selected from N, O, and S, or 5-6-membered heteroaryl with 1-2 heteroatoms selected from N, O, and S; and

Wherein $R_1$ is selected from H, halogen, CN, $NO_2$, $C_1$-$C_3$ alkyl, $C_2$-$C_3$ alkenyl, $C_2$-$C_3$ alkynyl, $C_5$-$C_6$ cycloalkyl, C5-C6 cycloalkenyl, halogenated $C_1$-$C_3$ alkyl, halogenated $C_2$-$C_3$ alkenyl, halogenated $C_2$-$C_3$ alkynyl, halogenated C5-C6 cycloalkyl, halogenated C5-C6 cycloalkenyl, ($C_6$-$C_8$)aryl, 5-6-membered heterocyclic group with 1-2 heteroatoms selected from N, O, and S, or 5-6-membered heteroaryl with 1-2 heteroatoms selected from N, O, and S.

**8.** The somatostatin analog of claim 1 or 4, wherein $X_2$ is a Lys derivative, as represented by the following:

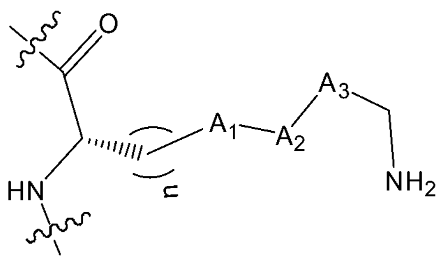

It is selected from the group consisting of:

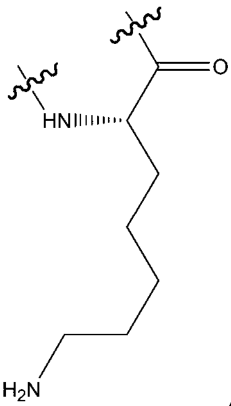 , 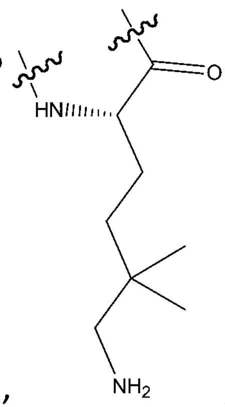 , 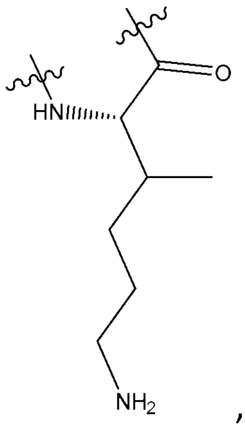 , 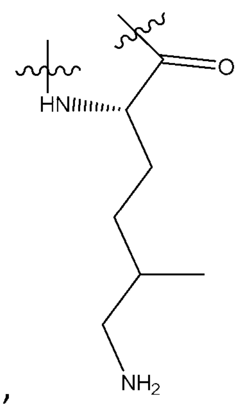 ,

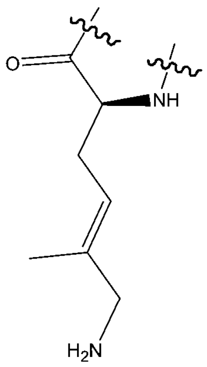 , 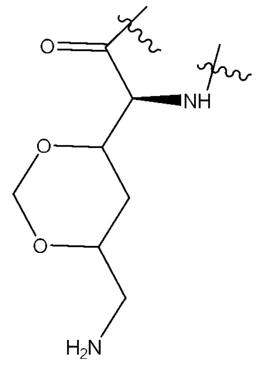 , 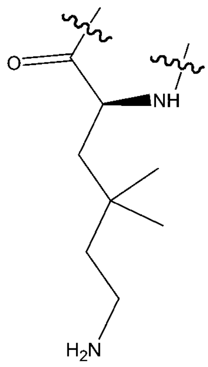 , 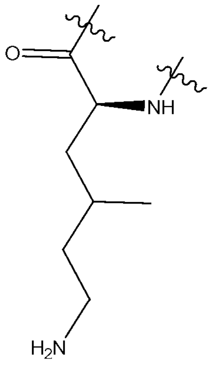 , 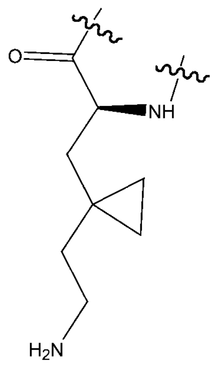 ,

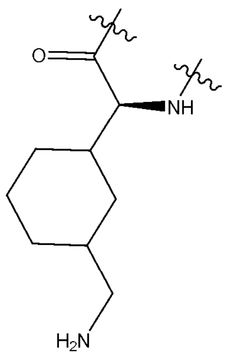 , 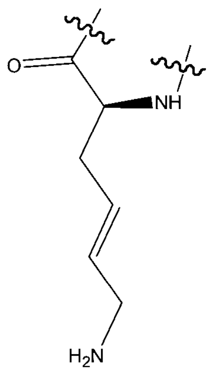 , 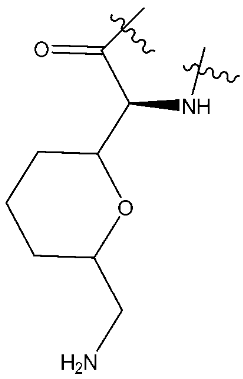 , 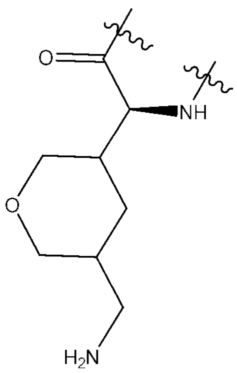 , 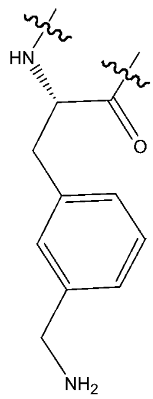 ,

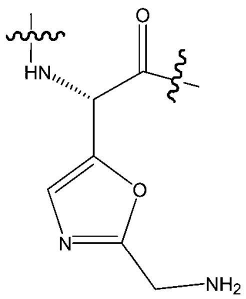 , 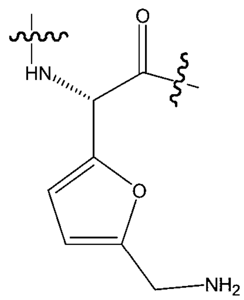 , 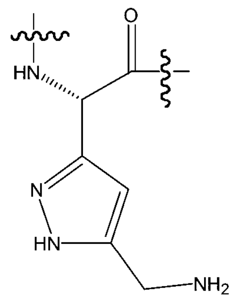 , 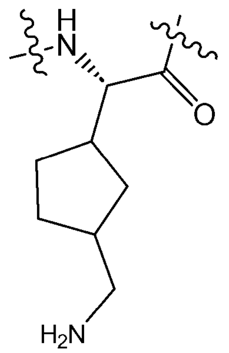 ,

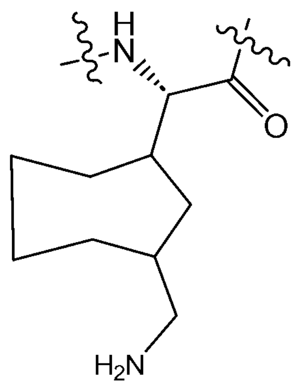 .

**9.** The somatostatin analog of claim 1 or 4, wherein the somatostatin analog is specifically selected from the group consisting of:

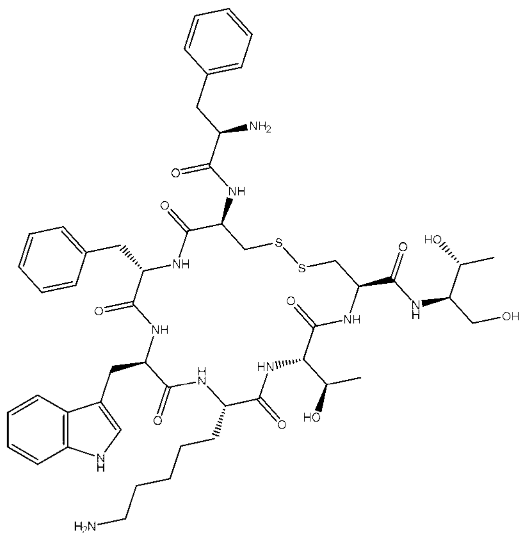

,

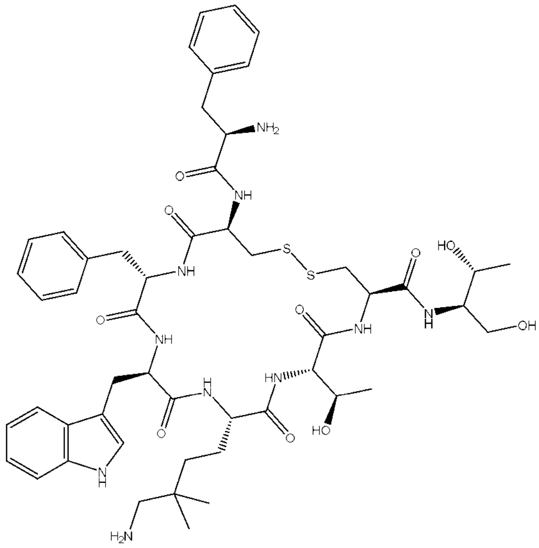

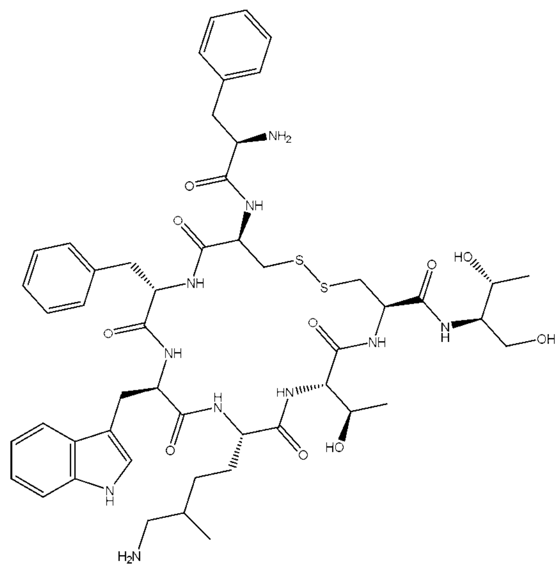

,

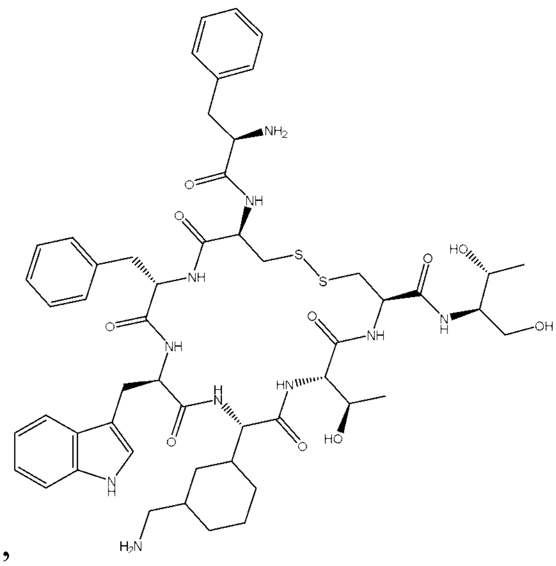

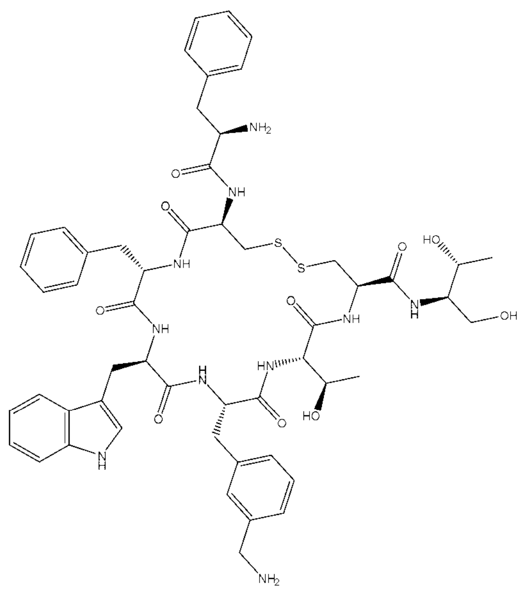

.

**10.** A somatostatin analog chelate, which is formed by removing the H atom from the N-terminal $NH_2$ of the somatostatin analog of claim 1 or 4 and the remaining NH either connects directly with a chelating group or indirectly via a linker.

**11.** The somatostatin chelate of claim 10, wherein the chelating group is selected from the group consisting of DTPA, DOTA, TETA, or substituted EDTA, and the optional linker is a divalent residue of aminocarboxylic acid, such as a β-Ala residue or a divalent residue derived from 6-aminocaprioic acid.

**12.** The use of the somatostatin analog of claim 1 or 4 in the preparation of anti-tumor drugs, preferably for the treatment of neuroendocrine tumors, acromegaly, and carcinoid tumors.

**13.** Salts, prodrugs, deuterated compounds, solvates, or hydrates of the somatostatin analog of claim 1 or 4.

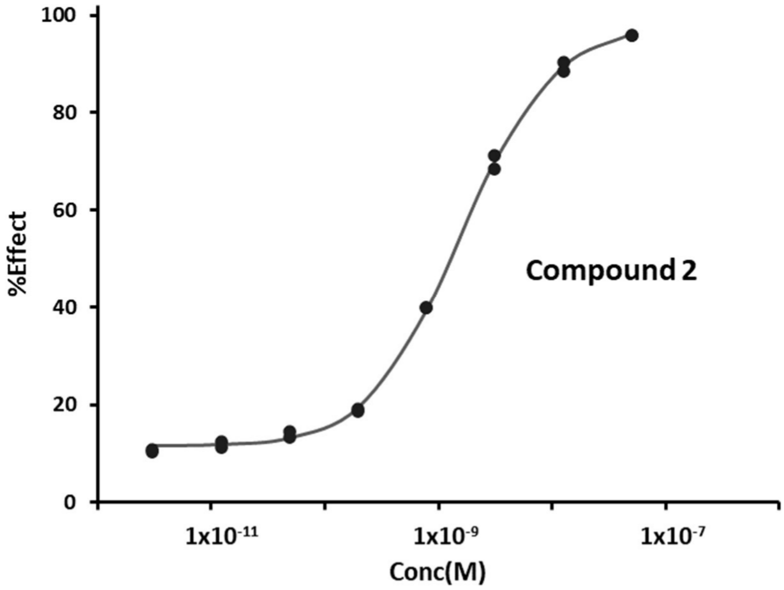
100
80
60
40
20
0
%Effect
Compound 2
1x10-11
1x10-9
1x10-7
Conc(M)

FIG. 3

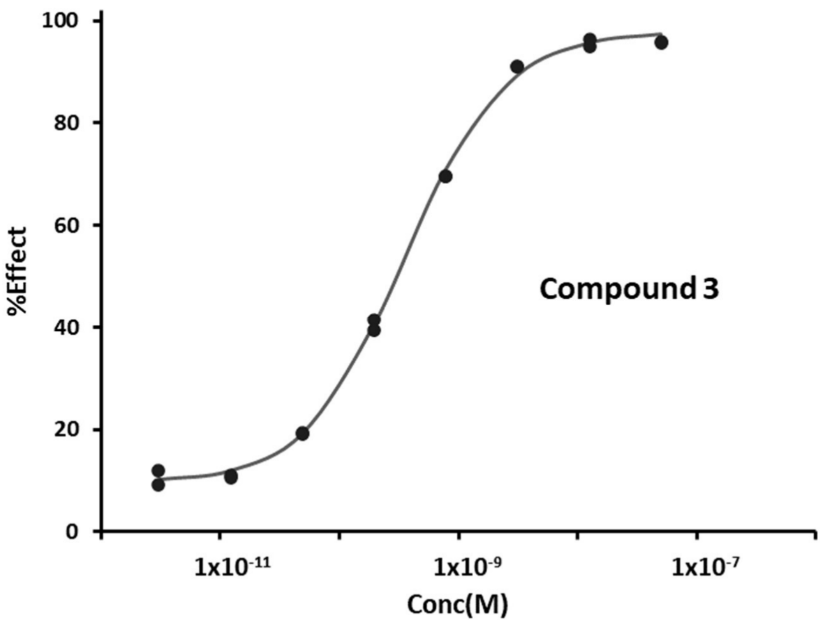
100
80
60
40
20
0
%Effect
Compound 3
1x10-11
1x10-9
1x10-7
Conc(M)

FIG. 4

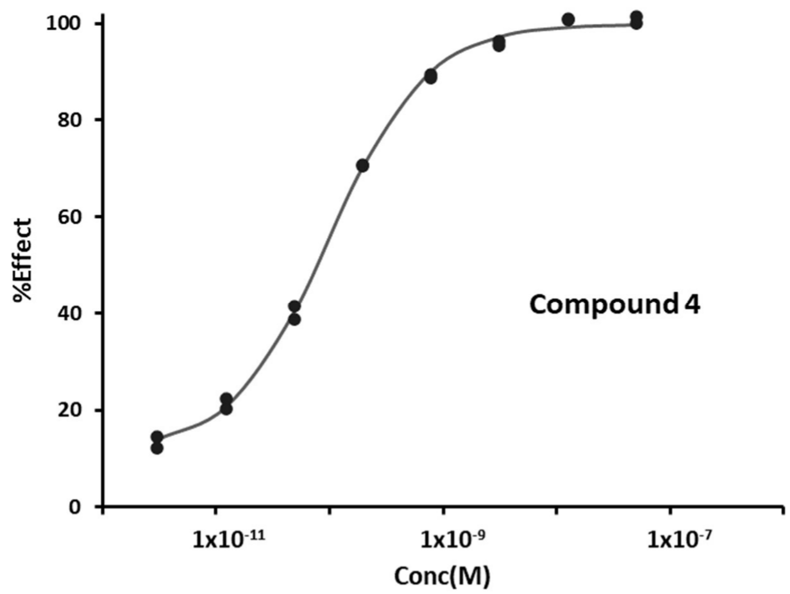
100
80
60
40
20
0
%Effect
Compound 4
1x10-11
1x10-9
1x10-7
Conc(M)

FIG. 5

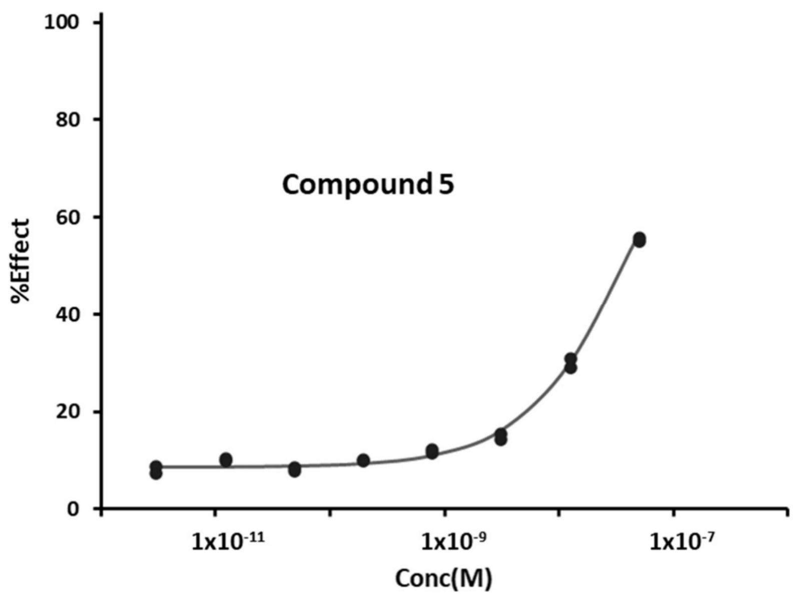
100
80
60
40
20
0
%Effect
Compound 5
1x10-11
1x10-9
1x10-7
Conc(M)

FIG. 6

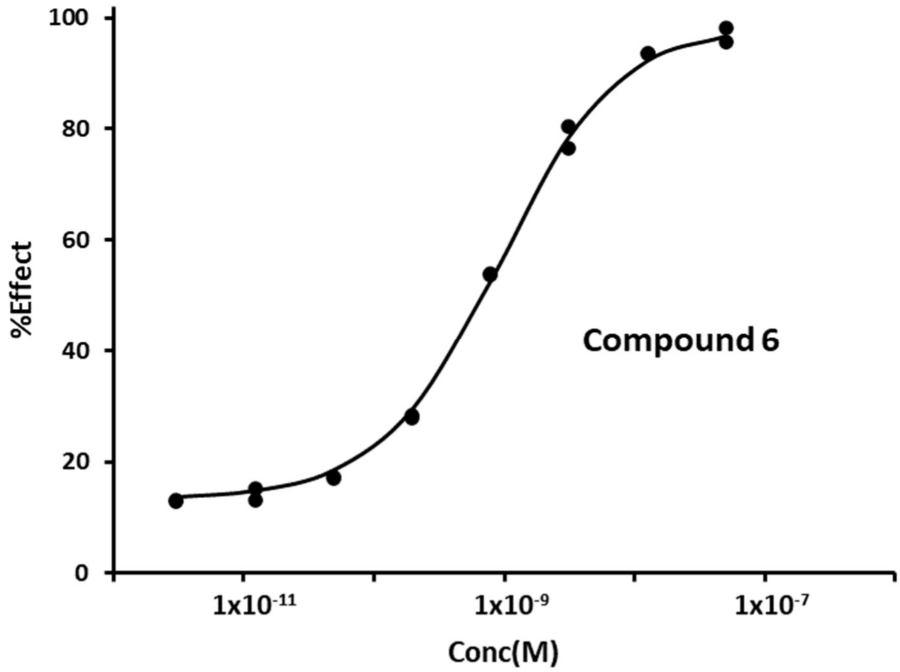
100
80
60
40
20
0
%Effect
Compound 6
1x10-11
1x10-9
1x10-7
Conc(M)

FIG. 7

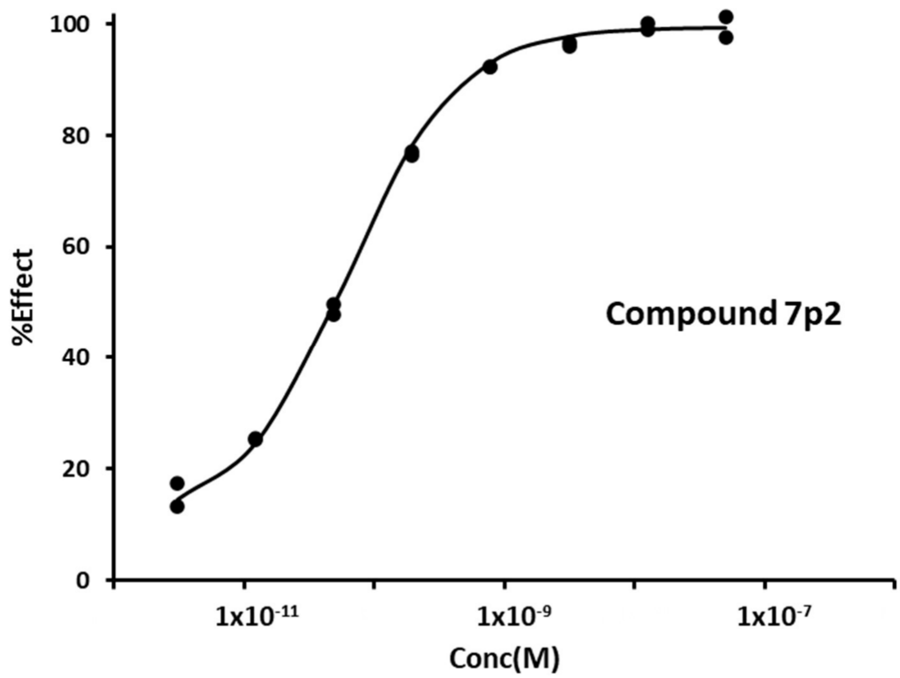
100
80
60
40
20
0
%Effect
Compound 7p2
1x10-11
1x10-9
1x10-7
Conc(M)

FIG. 8

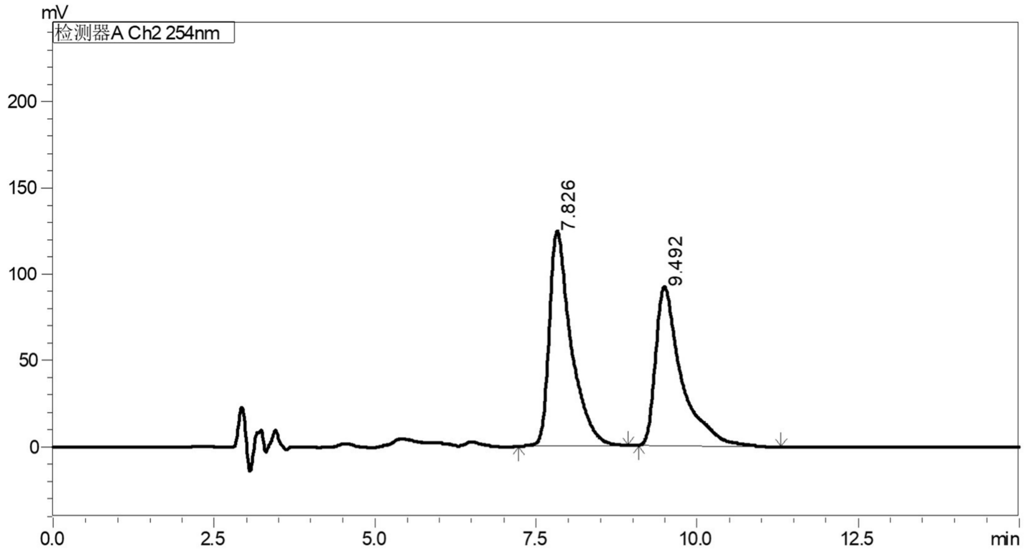
mV
检测器A Ch2 254nm
200
150
100
50
0
0.0
2.5
5.0
7.5
10.0
12.5
min
7.826
9.492

FIG. 9

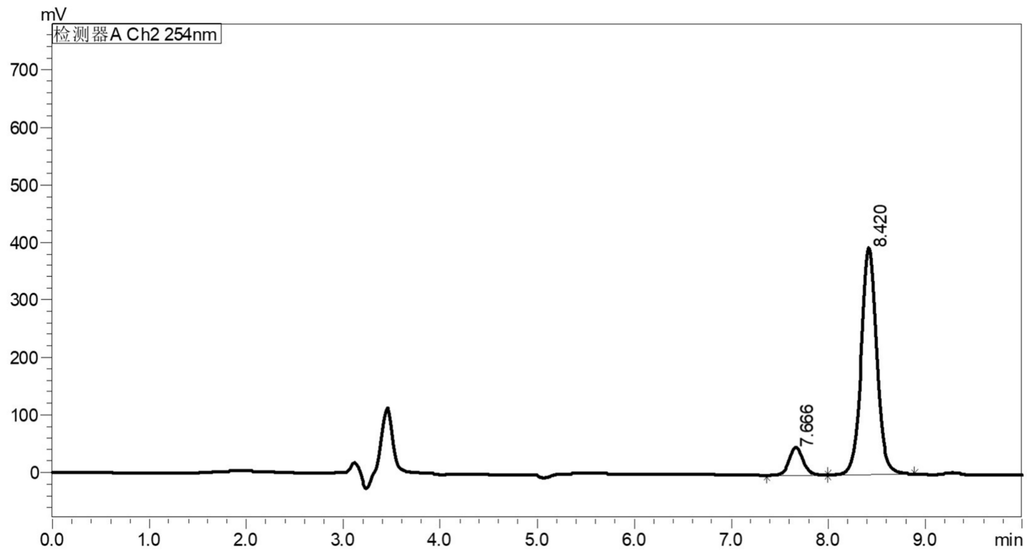
mV
检测器A Ch2 254nm
700
600
500
400
300
200
100
0
0.0
1.0
2.0
3.0
4.0
5.0
6.0
7.0
8.0
9.0
min
7.666
8.420

FIG. 10

## Brief Description of the Drawings

FIG. 1 is a graph illustrating the binding affinity of SSTR2 as a function of the concentration of the reference compound, Octreotide.

FIG. 2 is a graph illustrating the binding affinity of SSTR2 as a function of the concentration of Target Compound 1 (QHJ01-V001).

FIG. 3 is a graph illustrating the binding affinity of SSTR2 as a function of the concentration of Target Compound 2 (QHJ01-V017).

FIG. 4 is a graph illustrating the binding affinity of SSTR2 as a function of the concentration of Target Compound 3 (QHJ01-V002).

FIG. 5 is a graph illustrating the binding affinity of SSTR2 as a function of the concentration of Target Compound 4 (QHJ01-V029).

FIG. 6 is a graph illustrating the binding affinity of SSTR2 as a function of the concentration of Target Compound 5 (QHJ01-V003).

FIG. 7 is a graph illustrating the binding affinity of SSTR2 as a function of the concentration of Target Compound 6 (QHJ01-V059).

FIG. 8 is a graph illustrating the binding affinity of SSTR2 as a function of the concentration of Target Compound 7p2 (QHJ01-V29p2).

FIG. 9 is a chromatogram showing the separation of Compound 7h into 7h-p1+p2 and 7h-p3+p4.

FIG. 10 is a chromatogram showing the separation of Compound 7h-p1+p2 into 7h-p1 and 7h-p2.

## INTERNATIONAL SEARCH REPORT

International application No.
**PCT/CN2024/118116**

**A. CLASSIFICATION OF SUBJECT MATTER**

C07K14/655(2006.01)i; A61K38/31(2006.01)i; A61P35/00(2006.01)i; A61P5/00(2006.01)i; A61K38/08(2019.01)i

According to International Patent Classification (IPC) or to both national classification and IPC

**B. FIELDS SEARCHED**

Minimum documentation searched (classification system followed by classification symbols)

IPC:C07K; A61K; A61P

Documentation searched other than minimum documentation to the extent that such documents are included in the fields searched

Electronic data base consulted during the international search (name of data base and, where practicable, search terms used)

CNTXT, ENTXTC, CJFD, ENTXT, WFB, WFCCP, WFCD, WFCJ, WFCOA, WFCSTA, WFFCP, WFFD, WFFJ, WFFOA, WFS, WFSTR, PubMed, ISI Web of Knowledge, Baidu, CNKI: 钱杭江, 生长抑素, 抑生长素, 抑生长素肽, 拮抗剂, somatostatin, SSTR+, 赖氨酸, lys, 变异, 改造, 类似物, 修饰

**C. DOCUMENTS CONSIDERED TO BE RELEVANT**

| Category* | Citation of document, with indication, where appropriate, of the relevant passages | Relevant to claim No. |
|---|---|---|
| A | US 2008299040 A1 (SALK INSTITUTE FOR BIOLOGICAL STUDIES et al.) 04 December 2008 (2008-12-04)<br>claims 1-3 | 1-13 |
| A | CN 103288919 A (SALK INSTITUTE FOR BIOLOGICAL STUDIES et al.) 11 September 2013 (2013-09-11)<br>entire document | 1-13 |
| A | CN 105168115 A (CHINA PHARMACEUTICAL UNIVERSITY) 23 December 2015 (2015-12-23)<br>entire document | 1-13 |
| A | US 2020262871 A1 (YISSUM RESEARCH AND DEVELOPMENT COMPANY OF THE HEBREW UNIVERSITY OF JERUSALEM LTD.) 20 August 2020 (2020-08-20)<br>entire document | 1-13 |
| A | US 2010323964 A1 (VITALI, A. et al.) 23 December 2010 (2010-12-23)<br>entire document | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 December 2024** | **10 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/118116**

**C. DOCUMENTS CONSIDERED TO BE RELEVANT**

| Category* | Citation of document, with indication, where appropriate, of the relevant passages | Relevant to claim No. |
|---|---|---|
| A | CN 1189166 A (NOVARTIS AG) 29 July 1998 (1998-07-29) entire document | 1-13 |

Form PCT/ISA/210 (second sheet) (July 2022)

**INTERNATIONAL SEARCH REPORT**
**Information on patent family members**

International application No.
**PCT/CN2024/118116**

| Patent document cited in search report | Publication date (day/month/year) | Patent family member(s) | Publication date (day/month/year) |
|---|---|---|---|
| US 2008299040 A1 | 04 December 2008 | AU 2009236211 A1 | 22 October 2009 |
| | | AU 2009236211 B2 | 19 December 2013 |
| | | US 8691761 B2 | 08 April 2014 |
| | | RU 2010146497 A | 27 May 2012 |
| | | EP 2268667 A2 | 05 January 2011 |
| | | JP 2014129382 A | 10 July 2014 |
| | | WO 2009129311 A2 | 22 October 2009 |
| | | WO 2009129311 A3 | 22 July 2010 |
| | | BRPI 0910592 A2 | 22 September 2015 |
| | | BRPI 0910592 B1 | 19 October 2021 |
| | | CA 2721470 A1 | 22 October 2009 |
| | | CA 2721470 C | 07 March 2017 |
| | | EP 2801582 A1 | 12 November 2014 |
| | | EP 2801582 B1 | 06 September 2017 |
| | | ES 2646192 T3 | 12 December 2017 |
| | | JP 2011520787 A | 21 July 2011 |
| CN 103288919 A | 11 September 2013 | JP 2010506943 A | 04 March 2010 |
| | | JP 5295967 B2 | 18 September 2013 |
| | | CA 2666642 A1 | 24 April 2008 |
| | | CA 2666642 C | 17 November 2015 |
| | | SI 2076535 T1 | 31 July 2013 |
| | | PL 2076535 T3 | 30 August 2013 |
| | | DK 2076535 T3 | 10 June 2013 |
| | | EP 2383289 A1 | 02 November 2011 |
| | | EP 2383289 B1 | 08 October 2014 |
| | | EP 2433963 A1 | 28 March 2012 |
| | | EP 2433963 B1 | 04 June 2014 |
| | | BRPI 0717135 A2 | 15 October 2013 |
| | | BRPI 0717135 B1 | 15 September 2020 |
| | | BRPI 0717135 B8 | 25 May 2021 |
| | | ES 2526292 T3 | 09 January 2015 |
| | | AU 2007311137 A1 | 24 April 2008 |
| | | AU 2007311137 B2 | 20 June 2013 |
| | | ES 2410207 T3 | 01 July 2013 |
| | | PT 2076535 E | 12 June 2013 |
| | | WO 2008048942 A2 | 24 April 2008 |
| | | WO 2008048942 A3 | 18 September 2008 |
| | | US 2008260638 A1 | 23 October 2008 |
| | | US 7960342 B2 | 14 June 2011 |
| | | HK 1132515 A1 | 26 February 2010 |
| | | EP 2076535 A2 | 08 July 2009 |
| | | EP 2076535 B1 | 06 March 2013 |
| | | US 2011269683 A1 | 03 November 2011 |
| | | US 8501687 B2 | 06 August 2013 |
| | | PL 2433963 T3 | 28 November 2014 |
| | | PL 2383289 T3 | 31 March 2015 |
| CN 105168115 A | 23 December 2015 | None | |
| US 2020262871 A1 | 20 August 2020 | WO 2019058365 A1 | 28 March 2019 |
| | | US 11261215 B2 | 01 March 2022 |
| | | EP 3684794 A1 | 29 July 2020 |

Form PCT/ISA/210 (patent family annex) (July 2022)

**INTERNATIONAL SEARCH REPORT**
**Information on patent family members**

International application No.
**PCT/CN2024/118116**

| Patent document cited in search report | Publication date (day/month/year) | Patent family member(s) | Publication date (day/month/year) |
|---|---|---|---|
| US 2010323964 A1 | 23 December 2010 | KR 20100103464 A | 27 September 2010 |
| | | KR 101500528 B1 | 09 March 2015 |
| | | CY 1114490 T1 | 05 October 2016 |
| | | SI 2225271 T1 | 30 October 2013 |
| | | BRPI 0818978 A2 | 03 May 2016 |
| | | BRPI 0818978 B1 | 10 September 2019 |
| | | BRPI 0818978 B8 | 25 May 2021 |
| | | CA 2702940 A1 | 11 June 2009 |
| | | CA 2702940 C | 13 September 2016 |
| | | US 8937152 B2 | 20 January 2015 |
| | | ES 2431573 T3 | 27 November 2013 |
| | | EP 2225271 A2 | 08 September 2010 |
| | | EP 2225271 B1 | 31 July 2013 |
| | | PT 2225271 E | 27 September 2013 |
| | | DK 2225271 T3 | 07 October 2013 |
| | | PL 2225271 T3 | 31 December 2013 |
| | | JP 2011505345 A | 24 February 2011 |
| | | JP 5331817 B2 | 30 October 2013 |
| | | WO 2009071460 A2 | 11 June 2009 |
| | | WO 2009071460 A3 | 17 December 2009 |
| | | HRP 20130948 T1 | 08 November 2013 |
| CN 1189166 A | 29 July 1998 | AU 6515096 A | 30 January 1997 |
| | | AU 714447 B2 | 06 January 2000 |
| | | SK 177097 A3 | 05 August 1998 |
| | | SK 284087 B6 | 08 September 2004 |
| | | DK 0835263 T3 | 02 April 2002 |
| | | JP 2003104998 A | 09 April 2003 |
| | | KR 19990028480 A | 15 April 1999 |
| | | KR 100454664 B1 | 12 September 2005 |
| | | ATE 210152 T1 | 15 December 2001 |
| | | DE 69617687 D1 | 17 January 2002 |
| | | DE 69617687 T2 | 22 August 2002 |
| | | ES 2169251 T3 | 01 July 2002 |
| | | WO 9701579 A2 | 16 January 1997 |
| | | WO 9701579 A3 | 27 February 1997 |
| | | MY 147327 A | 30 November 2012 |
| | | TW 491854 B | 21 June 2002 |
| | | PT 835263 E | 29 April 2002 |
| | | TR 199701718 T1 | 21 May 1998 |
| | | NO 976064 D0 | 23 December 1997 |
| | | NO 976064 L | 16 February 1998 |
| | | NO 317867 B1 | 27 December 2004 |
| | | NZ 313147 A | 29 November 1999 |
| | | BR 9609335 A | 25 May 1999 |
| | | BR 9609335 B1 | 11 August 2009 |
| | | BR 9609335 B8 | 07 October 2014 |
| | | CZ 419697 A3 | 13 May 1998 |
| | | CZ 297381 B6 | 15 November 2006 |
| | | JPH 11506108 A | 02 June 1999 |
| | | JP 3445796 B2 | 08 September 2003 |

Form PCT/ISA/210 (patent family annex) (July 2022)

**INTERNATIONAL SEARCH REPORT**
**Information on patent family members**

International application No.
**PCT/CN2024/118116**

| Patent document cited in search report | Publication date (day/month/year) | Patent family member(s) | | | Publication date (day/month/year) |
|---|---|---|---|---|---|
| | | CA | 2222524 | A1 | 16 January 1997 |
| | | CA | 2222524 | C | 26 January 2010 |
| | | US | 6225284 | B1 | 01 May 2001 |
| | | PL | 323943 | A1 | 27 April 1998 |
| | | PL | 184947 | B1 | 31 January 2003 |
| | | IL | 122243 | A0 | 05 April 1998 |
| | | IL | 122243 | A | 25 September 2005 |
| | | EP | 0835263 | A2 | 15 April 1998 |
| | | EP | 0835263 | B1 | 05 December 2001 |
| | | EP | 0835263 | B9 | 24 July 2002 |
| | | HUP | 9901455 | A2 | 28 September 1999 |
| | | HUP | 9901455 | A3 | 28 November 2000 |
| | | HU | 228984 | B1 | 29 July 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2225579 A